# EUROPEAN PATENT SPECIFICATION

(11) **EP 0 880 360 B1**
(45) Date of publication and mention of the grant of the patent: **09.10.2002**
(21) Application number: 97903448.5
(22) Date of filing: 12.02.1997
(51) Int. Cl.: A61K 39/00, A61K 48/00

(54) **NOVEL METHODS OF VACCINATION AND VACCINES THEREFORE COMPRISING A NUCLEIC ACID ENCODING A FIRST EPITOPE AND A PEPTIDE CONTAINING A SECOND EPITOPE**
NEUE METHODE ZUR IMPFUNG SOWIE IMPFSTOFFE DAFÜR, DIE EINE EPITOPKODIERENDE NUKLEINSÄURE UND EIN EPITOPENTHALTENDE PEPTID BEINHALTEN
NOUVEAUX PROCEDES DE VACCINATION ET VACCINS ASSOCIES COMPRENANT UN ACIDE NUCLEIQUE CODANT UN PREMIER EPITOPE, AINSI QU'UN PEPTIDE CONTENANT UN SECOND EPITOPE

(30) Priority: 12.02.1996 GB 9602777; 30.04.1996 US 16506 P; 11.07.1996 GB 9614548; 16.08.1996 US 24116 P
(43) Date of publication of application: 02.12.1998
(73) Proprietor: Cobra Therapeutics Limited, Keele, Staffordshire ST5 5SP (GB)
(72) Inventor: CRAIG, Roger, Kingdon, Jubilee House Farm, Sanbach, Cheshire CW11 0XA (GB)
(74) Representative: Harrison Goddard Foote
(86) International application number: GB9700396
(87) International publication number: WO97028818

(56) References cited:
- WO-A-94/21806
- WO-A-95/05853
- WO-A-95/08635
- WO-A-96/41606
- NUCLEIC ACIDS RESEARCH, vol. 21, no. 9, 1993, pages 2065-2072, XP000652249 CARSON, S. AND M. V. WILES: "FAR UPSTREAM REGIONS OF CLASS II MHC Ea ARE NECESSARY FOR POSITION-INDEPENDENT, COPY-DEPENDENT EXPRESSION OF Ea TRANSGENE" cited in the application
- PROC. NATL. ACAD. SCI. USA, vol. 93, May 1996, pages 5141-5145, XP000652250 RAZ, E. ET AL.: "PREFERENTIAL INDUCTION OF A Th1 IMMUNE RESPONSE AND INHIBITION OF SPECIFIC IgE ANTIBODY FORMATION BY PLASMID DNA IMMUNIZATION" cited in the application

## Description

### Field of the Invention

The present invention relates to vaccines and methods of vaccination which involve transfection of cells.

### Background of the Invention

Vaccination has been an important medical pursuit ever since it was observed that, for certain diseases, initial exposure to the infectious agent conferred lifelong immunity against subsequent infections. Vaccines have been used for many years in order to build immunity in an individual against infection by particular pathogenic organisms such as viruses, bacteria, fungi and parasites.

Early vaccines relied on live organisms or killed organisms that retained their immunogenicity. A better understanding of the structure and function of particular pathogens and of the mechanisms of adaptive immunity has made it possible to design safer and more directed vaccines. The current vaccine against Hepatitis B virus, for example, relies on inoculation using only a portion of the viral surface antigen, rather than the complete organism. Such directed vaccines lead to fewer side effects and avoid unwanted immune responses to antigens that are not protective, i.e., do not confer lasting immunity.

Advanced vaccine design has made use of recombinant DNA technology and gene therapy concepts to provide DNA vaccines, wherein a vector for expression of a desired immunogen in mammalian cells is injected into a subject. The vector is taken up into cells around the site of injection, and then operates to express the immunogen in situ, which in turn leads to a protective immune response.

A problem associated with the use of DNA vaccines thus far is that the tissue into which the vector is typically injected, i.e., muscle, is not usually associated with antigen presentation. Consequently, the DNA vaccine does not produce a highly effective immune response.

There is a need for methods and specialized gene delivery vehicles suitable for delivery of complex antigens to cells so that the resulting antigen presentation is capable of activating substantially all of the components of the adaptive immune system, i.e., capable of eliciting the immune response necessary to combat a particular pathogen whether mediated by antibodies, cytotoxic T cells, helper T cells, natural killer cells, or macrophages.

### Summary of the Invention

The present invention provides methods and compositions for obtaining long-lasting immunity via delivery to an antigen presenting cell a complex comprising a nucleic acid encoding a first epitope, and a peptide containing a second epitope.

Antigen presenting cells present antigenic epitopes of antigens to T cells in association with either Class I or Class II MHC molecules. Endogenously synthesized proteins are generally presented in association with Class I whereas proteins taken up from the environment (exogenously synthesized proteins) are generally presented in association with Class II. It is therefore beneficial for a vaccine to produce presentation of the required antigen to both Class I and II MHC molecules. The invention provides for stimulation of both class I and II presentation by providing antigen to an antigen presenting cell in the form of an endogenously synthesized protein (i.e., a nucleic acid-encoded epitope) and an exogenously synthesized protein.

It is believed that the dual delivery to an antigen presenting cell of an epitope, or antigen, in its peptide or polypeptide form, together with a second nucleic acid encoded epitope results in an enhanced immune response.

The invention therefore encompasses a method of vaccinating a mammal against a disease, comprising administering to said mammal a mixture of (i) a nucleic acid encoding a first epitope and (ii) a peptide comprising a second epitope such that the nucleic acid and the peptide are taken up by and the nucleic acid is expressed in a professional antigen presenting cell of the mammal, wherein an immune response is elicited in the mammal to the epitopes.

The invention also encompasses a method of vaccinating a mammal against a disease wherein the mixture administered comprises a complex comprising (i) a nucleic acid encoding a first epitope and (ii) a peptide comprising a second epitope such that the complex is taken up by and expressed in a professional antigen presenting cell of the mammal, wherein an immune response is elicited in the mammal to the first and second epitopes.

The invention also encompasses a method of vaccinating a mammal against a disease, comprising administering to the mammal professional antigen presenting cells containing (i) a recombinant nucleic acid encoding a first epitope and (ii) a second epitope that is not normally present in the antigen presenting cells and, wherein upon administration an immune response is elicited in the mammal to the epitopes.

The invention also encompasses a composition for vaccinating a mammal against a disease, comprising a complex comprising (i) a vector comprising a nucleic acid encoding a first epitope and a sequence which permits maintenance of the vector in episomal form, and (ii) a peptide containing a second epitope, wherein said composition is adapted for delivery to or selective expression in antigen presenting cells.

The invention also encompasses a complex for vaccinating a mammal against a disease, comprising professional antigen presenting cells containing (i) a recombinant nucleic acid encoding a first epitope and (ii) a peptide containing a second epitope that is not normally present in the antigen presenting cells.

The invention encompasses a method of vaccinating a mammal against a disease, comprising administering to the mammal a complex comprising (i) a nucleic acid and (ii) a peptide including an epitope such that the complex is taken up by a professional antigen presenting cell of the mammal, wherein an immune response is elicited in the mammal to the epitope.

In this aspect of the invention, it is believed that the presence of the nucleic acid in the mixture promotes uptake by antigen presenting cells of the peptide containing an epitope so as to promote an immune response to the epitope.

Preferably, the first epitope is from an infectious agent or an organism, and the first epitope is present in the mammal during the course of a disease.

The complex further comprises a second epitope, and the first and second epitopes are epitopes of the same antigen, or epitopes of the same infectious agent or organism.

For example, the first and/or second epitope may comprise an immunodominant epitope of influenza NP.

It is also preferred that the complex or mixture further comprises a second peptide that contains an epitope that is different from the epitope contained in the peptide referred to in (ii) above.

The complex may further comprise a cell-targeting ligand for targeting professional antigen presenting cells.

It is preferred that the nucleic acid is intimately associated with the peptide containing the epitope such that the nucleic acid is preferably in condensed form. Therefore, it also is preferred that the first epitope is present in a two-domain polypeptide comprising the first epitope fused to a nucleic acid-binding amino acid sequence.

As used herein, the term "epitope" refers to an immunogenic amino acid sequence. An epitope may refer to a minimum amino acid sequence of 6-8 amino acids (i.e., a peptide), which minimum sequence is immunogenic when removed from its natural context and is carried in a complex according to the invention as a peptide, or when transplanted into a heterologous polypeptide such that it retains its natural immunogenicity and thus is carried in a complex according to the invention as part of a polypeptide. An epitope also may refer to that portion of a natural polypeptide which is immunogenic, where the natural polypeptide containing the epitope is referred to as an antigen. Of course, a polypeptide or antigen may contain one or more distinct epitopes. An epitope also may refer to an immunogenic portion of a multi-chain polypeptide, i.e., which is encoded by distinct open reading frames. The terms epitope, peptide, and polypeptide all refer to a series of amino acids connected one to the other by peptide bonds between the alpha-amino and alpha-carboxy groups of adjacent amino acids, and may contain or be free of modifications such as glycosylation, side chain oxidation, or phosphorylation, provided such modifications, or lack thereof, do not destroy immunogenicity. As used herein, the term "peptide" is meant to refer to both a peptide and a polypeptide or protein.

It is desirable that the epitope (peptide, polypeptide, antigen) be as small as possible while still maintaining immunogenicity. Immunogenicity is indicated by the ability to elicit an immune response, as described herein, for example, by the ability to bind an appropriate MHC molecule (i.e., an MHC class I or class II molecule) and to induce a T cell response and an antibody response, e.g., by measuring a cytotoxic T cell response or a serum antibody response to a given epitope(s).

As used herein, the terms "antigen" or "immunogen" refer to a peptide, protein, polypeptide which is immunogenic, that is capable of eliciting an immune response in a mammal, and therefore contains at least one and may contain multiple epitopes. According to the invention, a "pathogen", "organism", or "agent" may cause a disease for which vaccination is desired according to the invention. As used herein, these terms refer to a virus, bacteria, fungus, or a parasite. The term "agent" also may refer to antigens such as tumor antigens or antigens associated with autoimmunity. The invention does not contemplate administration of a whole pathogen, or its entire genome, to achieve antigenicity. Therefore, the term "epitope" is limited to this extent that it does not refer to a whole pathogen.

"Immune response" refers to either a cellular or a humoral immune response or to both a cellular and a humoral immune response.

It is believed that the antigenic peptide or protein delivered to the target cell by the delivery vehicle will be processed as an exogenously synthesized antigen and epitopes will be presented predominantly in association with Class II MHC molecules to Class II-restricted T cells. The antigen encoded by the nucleic acid of the delivery vehicle will be endogenously synthesized and epitopes of the antigen will be presented predominantly in association with Class I MHC molecules. Presentation in association with both MHC Class I and II molecules will ensure a strong immune response.

It is preferred according to the invention that the nucleic acid encoded antigen is expressed only in professional antigen-presenting cells (APC).

As used herein, the term "professional antigen-presenting cells" refers to MHC class II-bearing cells (that is, cells which express MHC class II or can be induced to express class II); such cells include B-lymphoctyes, dendritic cells other than follicular dendritic cells, macrophages, endothelial cells, phagocytic leukocytes, monocytes or monocyte derived cells, Kupffer cells, Langerhans cells, or stem cells thereof or other precursor cells, and other cells which either express class II or can be induced to express MHC class II.

The restricted expression of the gene to APCs or related cells can be achieved in two ways: 1) by targeting the delivery vehicle only to APCs or related cells and/or 2) by restricting the expression of the gene encoding the antigen to APCs or related cells.

It is contemplated according to the invention that the complex may further include a targeting ligand for targeting a receptor on the cell surface. Targeted delivery may be accomplished nonspecifically to both non-APCs and APCs, for example, where expression of the gene is restricted to APCs, or specifically to APCs only.

Thus, it is preferred that the complex of the present invention is adapted to target specifically to a professional antigen presenting cell of a mammal and/or the nucleic acid is adapted to be expressed specifically in a professional antigen presenting cell.

The term "to target specifically to a professional antigen presenting cell" refers to the situation where the complex is targeted to APCs and thus delivery will be substantially restricted to APCs. However, one skilled in the art will realize that targeting is rarely completely efficient and some delivery will occur to non-targeted cells. The term refers to where delivery to APCs is increased relative to other cell types, preferably by approximately two-fold or more.

Targeted delivery to APCs, their stem cells or other precursor cell types can be achieved by receptor-mediated gene transfer using delivery vehicles comprising the following examples of targeting ligands: (a) for hemopoietic stem cells: anti-CD34 monoclonal antibody, or the Stem cell factor (c-Kit or CD117), or flk-2 ligand (human homolog STK-1); (b) for monocyte/ macrophage/dendritic cell precursors: anti-CD33 monoclonal antibody; (c) for differentiated macrophage/dendritic cells: glycosylated DNA binding peptides carrying mannose groups may be used to target to specific receptors, for example the mannose receptor; and (d) for MHC class II bearing cells: an antibody that is specific for the constant region of MHC class II proteins or a ligand that binds MHC class II, for example soluble CD4; for example, one subset of MHC class II-bearing cells, B lymphocytes, may be targeted using soluble CD4 or using antibodies to or ligands for CD80, CD19, or CD22; for endothelial cells, γ-interferon and the vascular endothelial growth factor (VEGF) receptors; and (e) for APCs or T cells, ligands for or antibodies to co-stimulatory molecules such as B7-1, B7-2 or CD28, CTLA-4, respectively. These targeting ligands may play a dual role which involves increasing co-stimulatory signals to the APC, and thus increasing its activation, in addition to their targeting function.

Alternatively or in addition to the use of targeted complexes, DNA regulatory elements which lead to expression in APCs, their stem cells or other precursor cell types can be utilized.

The term "to be expressed specifically in a professional antigen presenting cell" refers to the situation where the expression of the nucleic acid is substantially restricted to APCs. The term therefore covers the situation where expression of the nucleic acid is restricted predominantly to APCs, for example, where approximately 50% and preferably 80%-100% of the cells in which expression of the nucleic acid occurs are APCs.

It is further contemplated according to the invention that the nucleic acid encoded antigen is predominantly expressed only in professional antigen-presenting cells (APCs).

Preferred regulatory elements include locus control regions (LCRs) such as the MHC class II LCR.

The present invention may be used to cause presentation of any protein or peptide capable of eliciting an immune response.

The present invention is applicable to any infectious agent against which an immune response can be measured. The invention also is applicable to other disease targets against which an immune response would be beneficial, such as tumors. Thus, the invention contemplates the use of a sequence encoding epitope of any infectious agent or disease target.

Preferably, the epitope is a key epitope, i.e., either the complete polypeptide sequence or an epitope which gives rise to a strong immune response to a particular infectious agent or disease. Examples of preferred epitopes include peptide epitopes of the influenza nucleoprotein (NP) gene, the tat, rev, gag and nef components of HIV, and epitopes from the E6 and E7 proteins of HPV. Additional preferred epitopes are those which are found to induce tolerance, such as the collagen involved in Rheumatoid arthritis.

More than one epitope may be encoded by the gene in order to increase the likelihood of an immune response. This is particularly important where the key epitopes which give rise to a protective immune responses have not been identified. Thus, in a further aspect of the present invention, the nucleic acid encodes more than one epitope capable of eliciting an immune response to a particular infectious agent or disease. This is particularly important where the key epitope(s) which give rise to a protective immune response have not been identified. Preferably, the epitope is present in a polypeptide (i.e., a polypeptide antigen) and therefore the polypeptide is encoded in a construct. Preferably, the construct is a multi-gene construct in which the antigens are co-ordinately expressed.

In a further embodiment of the present invention, where an immune response is desired to an infectious agent, all of the open reading frames from the pathogen's genome are arranged together in a construct in order to form the coding region of the delivery vehicle. This has the advantage that all the antigens will be present and thus the likelihood of an immune response increased.

Basically, an expression-library is made of the pathogen DNA and the expression-library delivered in the delivery vehicle to the cell in order to elicit an immune response.

In a further embodiment of the present invention, DNA sequences encoding antigens specific for different diseases or infectious agents may be arranged together in a construct for expression in the delivery vehicle of the present invention. The antigens may be whole proteins or multiple or single epitopes thereof arranged as a single gene or as a multi-gene construct. The delivery means will therefore provide immune protection against a number of diseases specified by the antigens encoded within the DNA construct.

In a further embodiment of the present invention the delivery vehicle may comprise mixture of antigenic peptide/protein components. These may be derived from the same antigen, they may be different antigens from the same infectious agent or disease, or they may be from different infectious agents or diseases. The complex or mixture will therefore raise an immune response against a number of antigens and possibly a number of infectious agents or diseases as specified by the antigenic peptide/protein components of the delivery system.

In a further embodiment of the present invention the antigenic components of the complex or mixture may, or may not, be glycosylated depending on the requirement for glycosylation for the generation of a suitable immune response against the epitope or antigen.

In a further embodiment of the present invention, the DNA encoded antigen or the antigenic component of the complex or mixture may not necessarily encode the most immunodominant epitopes of the antigen. They may be epitopes which are more highly conserved between different strains of an infectious agent or disease, or may be epitopes which have limited, or no, mutational variation. Antigens may also be encoded as a series of subgenes which cover the whole antigen but the whole antigen may not necessarily be encoded in a single gene.

In a further embodiment of the present invention the DNA may encode additional factors which will have the effect of upregulating the immune response to the encoded antigen, or protein/peptide component of the delivery system. The additional factors may include cytokines for the general upregulation of specific components of the immune response e.g. interferon gamma, IL-2, IL-4, IL-10, IL-12, and GM-CSF; lymphokines; or co-stimulatory molecules such as B7-1, B7-2, ICAM-1 and ICAM-3. Alternatively, each factor may be included in a mixture of complex according to the invention in its polypeptide form, in that it may be co-administered with a nucleic acid and antigen according to the invention or it may be conjugated to the antigen or to a nucleic acid binding peptide so as to form part of the delivery complex.

The invention encompasses the delivery of a vector or nucleic acid to a cell (i.e., the use of a delivery vehicle). As used herein, the phrase "means for delivering" a vector to a cell" or "adapted for delivery" to a cell refers to any means, including viral and non-viral delivery means, by which it is possible to deliver nucleic acid and an antigenic peptide or protein associated with nucleic acid to a mammalian cell, including DNA/polycation complexes, self assembling virus like particles, viral vectors which are capable of delivering nucleic acid to a mammalian cell such as adenoviruses, retroviruses and adeno-associated viruses, microspheres which are used for delivery of DNA or protein to cells, e.g., polylactide glycolide polymers, and liposomes. Delivery means useful according to the present invention are well known to those skilled in the art and are described further herein.

Particularly preferred delivery vehicles are those which include polycation-condensed nucleic acid which in addition may be coupled with a ligand for targeting cells specifically or non-specifically.

The delivery vehicle may therefore include a nucleic acid condensing peptide, e.g., a polycationic heteropeptide, which binds DNA (i.e., a DNA binding peptide).

In another embodiment of the invention, the complex or mixture may include a peptide which does not necessarily participate in delivery of the vector or nucleic acid to the cell, but which is antigenic and thus serves to induce an immune response in a mammal upon entry of the delivery vehicle into the cell. The antigenic peptide may be a portion of a two-domain peptide comprising a DNA-binding amino acid sequence and the antigenic peptide. The two domains may be fused via a peptide bond to form a fusion polypeptide, wherein the antigenic domain is amino terminal and the DNA binding domain is carboxy terminal, or wherein the DNA binding domain is amino terminal and the DNA binding domain is carboxy terminal. Where a protein contains a DNA binding domain and also an epitope(s) to which an immune response is desired, the protein may be considered to be essentially equivalent to a two domain peptide as described herein.

Alternatively, the two domains may be separated by a cleavable linker such as a acid labile linkage or a peptide sequence cleavable by an endosomal protease such as cathepsin. In another embodiment, it is also contemplated that the epitope is not part of a fusion protein but is directly absorbed onto a complex comprising a nucleic acid encoding an epitope in association with a DNA binding domain by electrostatic, hydrophobic, covalent or other interactions.

Where a DNA binding peptide is used in a complex or mixture of the invention, whether it be as part of a two domain fusion polypeptide or noncovalently associated with an epitope, it is envisioned that the DNA binding peptide binds the nucleic acid of the complex or mixture in a condensing reaction and therefore permits intimate association of the epitope portion of the complex or mixture with the nucleic acid.

In a further embodiment of the present invention the antigen encoding nucleic acid sequence may contain a signal sequence for secretion of the antigen outside the cell. Signal sequences useful in the present invention are well known to those skilled in the art and are, for example, described in Blobel and Dobberstein (1975), J. Cell Biol., 67, 852-862. The secreted antigen will be taken up by the secreting cell and other neighbouring cells, processed as an exogenous antigen, and presented in association with Class II MHC. The delivery vehicle of the present invention may contain a mixture of nucleic acids encoding an antigen, some with, and some without, the secretion signal sequence. Thus secreted and non-secreted antigen can be produced in the same cell and both Class I and Class II MHC presentation can be produced after a single transfection event.

An antigenic peptide useful as an antigen component of a delivery vehicle or in a two-domain peptide may be from the same or a different organism against which a DNA-based immune response is desired. An example of a preferred antigenic peptide for this purpose is the immunodominant peptide epitope(s) of influenza nucleoprotein (NP).

Upon entry of this delivery vehicle into a cell, an immune response will be elicited to the antigenic peptide. The immune response will be subsequently sustained and expanded by the expression of the antigenic protein encoding sequences also present in the delivery vehicle.

It is further preferred that the vector containing a nucleic acid encoding an antigen is maintained at a high copy number in dividing and non-dividing cells of a patient.

Thus, particularly preferred vectors useful according to the invention contain a sequence which permits maintenance of the vector in an episomal form. Such vectors may comprise a minimal origin of replication of a papilloma virus, a minichromosomal maintenance element of a papilloma virus, and a cloning site for inserting a nucleic acid encoding one or more antigens. This may be achieved by employing the BPV-I vector system comprising a plasmid harboring the BPV-1 origin of replication, or a minimal origin plus minichromosomal maintenance element, as disclosed hereinbelow, and optionally the BPV-1 E1 and E2 genes.

If desired, the antigenicity of selected proteins (e.g., targeting ligands) or peptides of the delivery vehicle, or other proteins encoded by the vector, (e.g., the BPV-1 E1 and E2 proteins) may be eliminated by introducing into their open reading frames sequences from the Epstein bar virus EBNA-1 protein encoding a Gly-Ala repeat, that suppresses antigen presentation of amino acid sequences linked in cis.

A composition of the present invention may be used in *ex vivo* gene therapy or in *in vivo* gene therapy in order to provide immune protection against various infectious agents and diseases.

### Brief Description of the Drawings

The present invention is illustrated in the appended examples, with reference to the following figures:

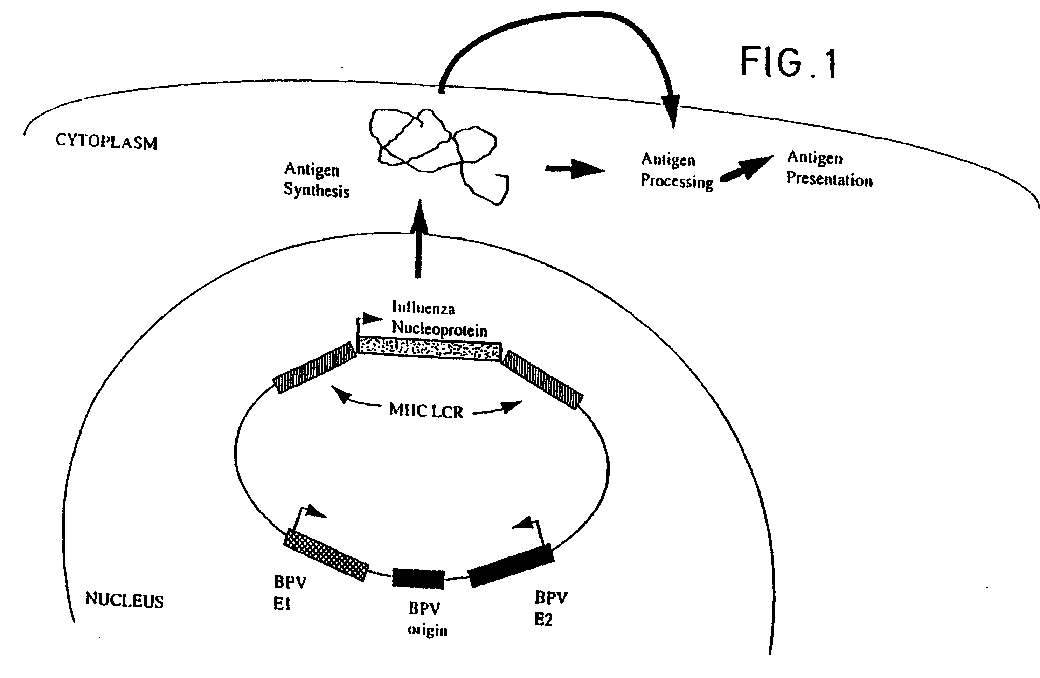

Figure 1 is a schematic diagram showing sustained, targeted expression of antigenic proteins in professional antigen presenting cells (dendritic cells).

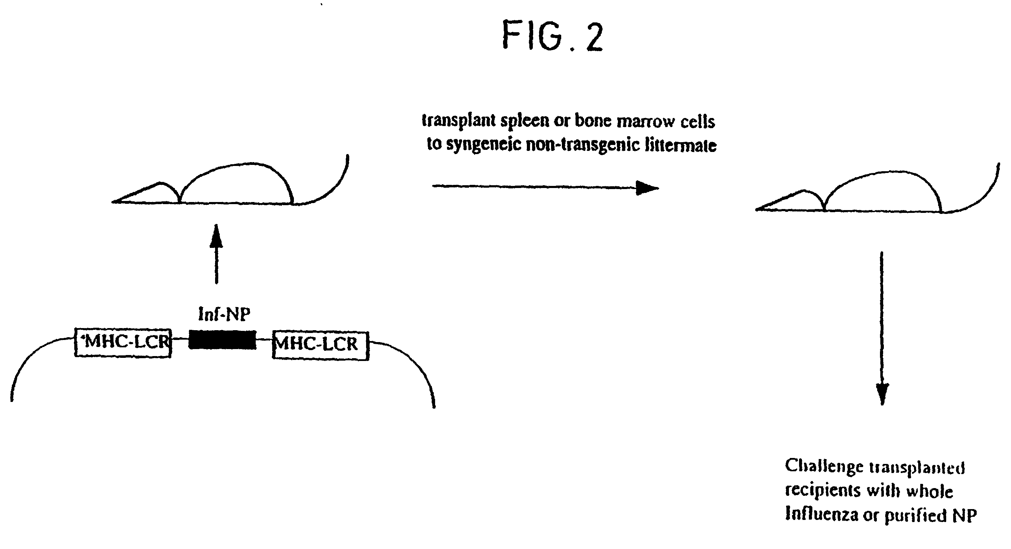

Figure 2 is a schematic diagram showing *in vivo* vaccination against infectious agents where gene expression in professional antigen presented cells.
Figure 3 demonstrates cell-specific expression of the human glucocerebrosidase gene under the control of the MHC class II LCR in APCs.

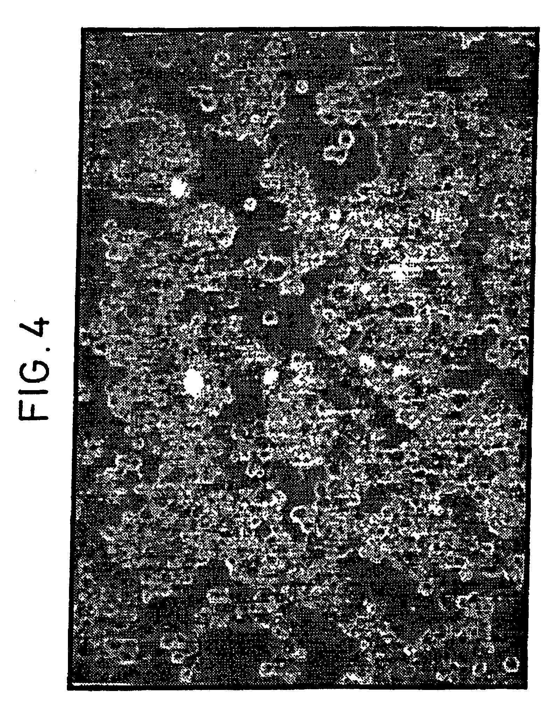

Figure 4 is a microscopic view of an in vitro matured dendritic cells transfected with pEGFP-N1. Transfected cells are fluorescent.
Figure 5 represents FACScan analysis of COS cells incubated with either buffer (A), peptide alone (B), or complex (C). D represents an overlay plot (thin line, buffer; solid broad line, peptide, lined line, complex).
Figure 6 is a schematic illustration of the plasmid pCMVluc, a luciferase expression vector which has the luciferase gene driven from the CMV immediate early promoter. The plasmid includes the luciferase gene, which contains an SV40 intron and polyA sequence, a bacterial origin of replicaton and the beta-lactamase gene for selection in bacteria. The plasmid also includes the puromycin gene for selection in eukaryotic cells.
Figure 7A presents data for triplicate transfections of COS cells with naked DNA or K6ClII and DNA complexes. The values provided are: luminescence units read from a luminometer (lum units); the amount of protein found in the sample (mg); luminescence units per mg protein (1um/mg); average luminescence units per mg of protein each each condition (Av); standard error (St. Error).
Figure 7B is a bar graph of the data presented in Fig.. 7A.

### Description

The invention is based on the observation that an immune response to an antigen may be rendered highly specific and effective by delivery to antigen presenting cells of a mixture comprising an antigen in its peptide or polypeptide form and a nucleic acid encoding an antigen.

The invention also is based on the observation that an immune response to an antigen may be rendered highly effective by delivery to antigen presenting cells of a complex comprising an antigen in its peptide or polypeptide form and a nucleic acid encoding an antigen.

The invention also is based on the observation that an immune response to an antigen may be rendered highly effective by delivery to antigen presenting cells of a complex comprising an antigen in its peptide or polypeptide form and a nucleic acid. In this aspect of the invention, the nucleic acid need not encode an antigen, but is useful to promote uptake of the antigen by the antigen presenting cell.

In the simplest form, the peptide antigen and the nucleic acid encoded antigen described herein are the same. However, it is believed that a more effective immune response may be obtained using a first peptide antigen in combination with a second different nucleic acid-encoded antigen, or wherein several different peptide antigens are administered in combination with one or several different nucleic acid-encoded antigens. A "more effective" immune response will be evident, as it relates to prior art vaccination procedures and compositions, as a two-fold and preferably a five-fold to ten-fold higher immune response, or by the finding that both a cellular and a humoral immune response is elicited by complexes or mixtures of the invention. Methods and compositions for making and carrying out the invention are described in detail below.

### Vaccination via Antigen Presenting Cell-Restricted Gene Expression According to the Invention

The invention provides for vaccination against a disease or pathogen via administration to a mammal of a mixture or complex described according to the invention and, in some cases, of antigen presenting cell- (i.e., tissue-) restricted expression of a nucleic acid encoding an antigen associated with the disease or pathogen. Tissue-restricted expression of a gene encoding an antigen for which an immune response is desired, wherein the tissue to which expression is restricted comprises antigen presenting cells, as defined herein, or subsets thereof is obtained as follows.

Two modes of effective antigen presenting cell-restricted gene expression are contemplated according to the invention: 1) via targeted delivery of a mixture or complex comprising an antigenic peptide and a gene (preferably encoding an antigen for which an immune response is desired) to antigen presenting cells, and 2) via either targeted or untargeted gene delivery (and thus possible delivery of the gene to different cell types) wherein control of expression of the gene is effected using genetic control elements which limit gene expression to the desired antigen presenting cells or subset of cells.

### Delivery of Nucleic Acid to Host Cell

It is contemplated according to the invention that a mixture or complex according to the invention may be delivered to an antigen presenting host cell non-specifically or specifically (i.e., to a designated subset of host cells). Three modes of delivery are contemplated. First, wherein no targeting ligand is used. Second and third, where a targeting ligand is employed; in the case of non-specific delivery to cells, a non-specific ligand is used that targets a cell surface receptor that is present on the target cell population as well as on other cells; in the case of specific delivery, a ligand is used that targets a specific subset of cells. Therefore, the complex or mixture may be delivered generally to any cell, or may be delivered to antigen presenting cells, or may be delivered to a subset of antigen presenting cells, these subsets and targeting ligands and cognate receptors being described herein.

### 1. Non-Viral Delivery to APCs

Non-viral delivery to APCs may or may not employ a targeting ligand. Targeted delivery to APCs, their stem cells or other precursor cell types can be achieved by receptor-mediated gene transfer using a complex containing a ligand which is targeted to a cognate receptor on a cell surface. Targeting ligands useful according to the invention include but are not limited to the following: (a) for hemopoietic stem cells: anti-CD34 monoclonal antibody, or the Stem cell factor (c-Kit or CD117), or flk-2 ligand (human homolog STK-1) ; (b) for monocyte/macrophage/dendritic cell precursors: anti-CD33 monoclonal antibody; (c) for differentiated macrophage/ dendritic cells: glycosylated DNA binding peptides carrying mannose groups may be used to target to specific receptors, for example the mannose receptor; and (d) for MHC class II bearing cells: an antibody that is specific for the constant region of MHC class II proteins or a ligand that binds MHC class II, for example soluble CD4; for example, one subset of MHC class II-bearing cells, B lymphocytes, may be targeted using soluble CD4 or using antibodies to or ligands for CD80, CD19, or CD22; for endothelial cells, γ-interferon; and (e) for APCs or T cells, co-stimulatory molecules such as B7-1, B7-2 or CD28, CTLA-4, respectively.

Targeted delivery vehicles for delivery of DNA constructs to cells are known in the art and include DNA/polycation complexes which are specific for a cell surface receptor, as described in, for example, Wu and Wu (1988) *J. Biol. Chem* 263:14621; Wilson et al. (1992) *J. Biol. Chem*. 267:963-967; and U.S. Patent No. 5,166,320, and, for example, USSN 60/011,531, assigned to the same assignee. In this co-pending application, a self-assembling virus-like particle is described and includes the DNA of interest and condensing peptides which are heteropeptides with respect to their amino acid composition (i.e., containing at least two different amino acids which are preferably basic and thus good DNA binding and DNA condensing peptides) and which have low polydispersion (i.e., a given preparation of a heteropeptide which has low polydispersion contains peptides of very similar, if not identical lengths, such that the preparation is essentially monodispersed).

The invention thus also relates to a nucleic acid construct which is delivered to a cell using a synthetic virus like particle for transfecting nucleic acid into a mammalian cell. The synthetic virus like particle includes a recombinant nucleic acid, a plurality of nucleic acid condensing peptides, the peptides being non-covalently associated with the recombinant nucleic acid such that the nucleic acid is in condensed form, wherein each nucleic acid condensing peptide is a heteropeptide, and plurality of nucleic acid condensing peptides has low polydispersion.

The plural nucleic acid condensing peptides may include a first nucleic acid condensing peptide and a second nucleic acid condensing peptide, wherein the first nucleic acid condensing peptide comprises a first functional group covalently bound thereto. The first nucleic acid condensing peptide may further include a second functional group which may be directly bound to the peptide or may be covalently bound to the first functional group, where the first functional group is bound to the peptide. Alternatively, a second nucleic acid condensing peptide also may include a second functional group covalently bound thereto, the second functional group being different from the first functional group. The first and second nucleic acid condensing peptides may have identical or different amino acid sequences.

The functional groups which are bound to peptides useful according to the invention include antigenic peptides or proteins, such as influenza nucleoprotein (NP), or a ligand that targets a specific cell-type such as a monoclonal antibody, insulin, transferrin, asialoglycoprotein, or a sugar. The ligand thus may target cells in a non-specific manner or in a specific manner that is restricted with respect to cell type.

The first nucleic acid condensing peptide may include 8-24 positively charged amino acid side groups; for example, the number of positively charged amino acid side groups may be in the range of 12-18.

The ratio of positive/negative charges in a synthetic virus like particle that is capable of targeting a specific mammalian cell type is within the range 0.5-3 per phosphate residue in the nucleic acid; this ratio thus also may be within the range 0.8 - 1.2.

The ratio of positive/negative charges in a synthetic virus like particle that is unrestricted with respect to the type of cell it targets is in within the range of 0.5 - 5 per phosphate residue in the nucleic acid, and thus also may be within the range of 1.2 - 2.

A nucleic acid condensing peptide which is particularly useful for condensing the nucleic acid construct and therefore for delivering nucleic acid to a cell includes a peptide of the generic formula

NH₂-A-(X₁X₂Y₁Y₂)ₙX₃X₄-(Z₁Z₂Z₃Z₄) -(X₅X₆Y₃Y₄)ₘX₇X₈BCOOH

wherein each of X₁₋₈ is, independently, an amino acid having a positively charged group on the side chain; wherein each of Y₁-₄ is, independently, a naturally occurring amino acid which promotes alpha helix formation; wherein each of Z₁₋₄ is, independently, a naturally occurring amino acid with at least 3 amino acids having a high propensity to form a stabilized turn structure; wherein A is an amino-terminal serine or threonine residue; wherein B is any amino acid; and wherein n = 2 - 4 and m = 2.

Other peptides are those wherein each of X₁₋₈ is, independently, lysine, arginine, 2.4-diamino-butyric acid or ornithine; wherein each of Y₁-₄ is, independently, glutamic acid, alanine, leucine, methionine, glutamine, tryptophan or histidine; wherein each of Z₁₋₄ is, independently, asparagine, glycine, proline, serine, and aspartic acid; wherein B is any one of alanine, glutamic acid or cysteine.

It is also contemplated according to the invention that peptides useful in this embodiment of the invention which involves delivery of a complex according to the invention to a cell either ex vivo or in vivo may contain one or more internal Serine, Threonine, or Cysteine residues, preferably at a position in the sequence which will be exposed for conjugation to a selected ligand, and thus not on the positively charged (nucleic acid oriented) face of the α-helix. This positioning of selected reactive amino acid residues within the peptide are oriented such that they do not contact the face of the peptide that contacts nucleic acid permits conjugation of the peptide with other functional peptides by bonds of selected and defined stability. Cysteine allows specific conjugation via the thiol side chain to compounds containing other reactive thiol groups (via disulfides), alkylating functions (to form thioether bonds), or other thiol reactive groups such as maleimide derivatives.

Peptides which fall within this generic sequence include:

NBC7 TRRAWRRAKRRAARRCGVSARRAARRAWRRE-OH; and
NBC11 H-TKKAWKKAEKKAAKKCGVSAKKAAKKAWKKA-NH2.

Thus, a nucleic acid condensing peptide useful for delivery of a nucleic acid may contain: 1) helix-forming amino acids, 2) a repeating three-dimensional structure that contacts the major groove of the nucleic acid, 3) suitable chromophores for quantitation, and 4) a number of "handles" (i.e., reactive sites) for regio-specific conjugation of ligands which form accessory functional domains.

Nucleic acid condensing peptides also may include portions of H1 (sequence I, II or III below) which are identified herein as sequences which possess the ability to condense nucleic acid. Therefore, a nucleic acid condensing peptide can include a linear combination of the following three consensus sequences where the total sequence length is >17 residues: Sequence I:   -K-K-X-P-K-K-Y-Z-B-P-A-J- where: K is Lysine, P is Proline; A is Alanine; X is Serine, Threonine or Proline; Y is Alanine or Valine; Z is Alanine, Theonine or Proline; B is Lysine, Alanine, Threonine or Valine; and J is Alanine or Valine. Sequence II:    -X-K-S-P-A-K-A-K-A- where: X is Alanine or Valine; K is Lysine; S is Serine; P is Proline; and A is Alanine. Sequence III: -X-Y-V-K-P-K-A-A-K-Z-K-B- where: X is Lysine or Arginine; Y is Alanine or Threonine; Z is Proline, Alanine or Serine; B is Lysine, Threonine or Valine; K is Lysine; P is Proline; A is Alanine.

One such peptide is NBC1, which has the following structure:
NH₂-[SV40 NLS]-[Seq I]-[Seq II]-[Seq III]-[SV40 NLS]-[Seq I]-C-COOH, where -C- is Cysteine; where the SV40 NLS has the sequence Pro-Lys-Lys-Lys-Arg-Lys-Val-Gln; and the sequence H-PKKKRKVEKKSPKKAKKPAAKSPAKAKAKAVKPKAAKPKKPKKKRKVEKKSP KKAKKPAAC(Acm)-OH.

Another such nucleic acid condensing peptide of the invention will have a peptide that falls within the following generic sequence:
NH₂-X-(Y)ₙ-C-COOH, where X is either absent or Serine or Threonine; Y is sequence I, II or III as defined above; n is 2-6; and C is Cysteine.

Other such peptides have the following structures and sequences: NBC2 has the structure: NH₂-[Seq III]-[SV40 NLS1]-[Seq I]-C-COOH, where -C- is Cysteine.
NBC8 has the structure: NH₂-[Seq I]-[Seq I]-C-COOH, where -C- is Cysteine.
NBC9 has the structure: NH₂-[Seq I]-[Seq I]-[Seq I]-C-COOH, where -C- is Cysteine.
NBC10 has the structure: NH₂-[Seq I]-[Seq I]-[Seq I]-[Seq I]-C-COOH
where -C- is Cysteine; the amino acid sequences of which are as follows:

NBC2 H-KAVKPKAAKPKKPKKKRKVEKKSPKKAKKPAAC(Acm)-OH;
NBC8 H-KKSPKKAKKPAAKKSPKKAKKPAAC(Acm)-OH;
NBC9 H-KKSPKKAKKPAAKKSPKKAKKPAAKKSPKKAKKPAAC(Acm)-OH;
NBC10 H-KKSPKKAKKPAAKKSPKKAKKPAAKKSPKKAKKPAAKKSPKKAKKP
AAC(Acm)-OH.

As described above, nucleic acid condensing peptides having a low polydispersion index (PDI) are useful for delivery to a cell of a nucleic acid encoding an antigen according to the invention. The PDI for such peptides may be calculated from analysis of the peptides by electrospray mass spectrometry. This method gives the exact mass of each component to within 0.001%. The PDI values of the peptide preparations useful in the present invention are in the range of 1.0 - 1.100. Peptide preparations which are especially useful in the invention possess a PDI <1.01, and even <1.001.

Preferred delivery vehicles are prepared as follows. A synthetic virus like particle is formulated such that the nucleic acid encoding an antigen and the peptide preparation are prepared in equal volumes of the same buffer. The nucleic acid is shaken or vortexed while the condensing peptide preparation is added at the rate of 0.1 volume per minute. The complex is left at room temperature for at least 30 minutes prior to addition to the target cells or prior to administration to a subject, and can be stored at 4°C. The particle is centrifuged to remove any aggregated material.

In addition to the above-described DNA/polycation complexes for cell targeting, methods are known in the prior art for preparing cell-targeting liposomes containing nucleic acid. An example of targeting liposomes is immunoliposomes, which are prepared, for example, by adsorption of proteins (e.g., immunoglobulin) on the liposomal surface; incorporation of native protein into the liposome membrane during its formation (e.g., by ultrasonication, detergent dialysis or reverse phase evaporation); covalent binding (direct or via a spacer group) of a protein to reactive compounds incorporated into the liposomes membrane; noncovalent hydrophobic binding of modified proteins during liposome formation or by the incubation with preformed liposomes); and indirect binding, including covalent binding of immunoglobulin protein via a polymer to the liposome (see Torchilin, V.P. CRC Critical reviews in Therapeutic Drug Carrier Systems, vol. 2(1)). Binding of the nucleic acid-ligand complex to the receptor facilitates uptake of the nucleic acid by receptor-mediated endocytosis.

A nucleic acid-ligand complex linked to adenovirus capsids, which naturally disrupt endosomes, thereby releasing material into the cytoplasm, can be used to avoid degradation of the complex by intracellular lysosomes (see for example Curiel et al. (1991) *Proc. Natl. Acad. Sci. USA* 88:8850; Cristiano et al. (1993) *Proc. Natl. Acad. Sci. USA* 90:2122-2126).

Receptor-mediated nucleic acid uptake can be used to introduce nucleic acid into cells either *in vitro* or *in vivo* and, additionally, has the added feature that nucleic acid can be selectively targeted to a particular cell type by use of a ligand which binds to a receptor selectively expressed on a target cell of interest, or can be non-selective with respect to the target cell type.

The precise stoichiometric ratio of the various components of the complex or mixture according to the invention can be varied in order to control the magnitude of the initial immune response, the efficiency of delivery and the degree of specific targeting to APCs or related cells. Generally, the ratio of the nucleic acid encoding a first epitope to the amino acid sequence encoding the second epitope will be in the range of 1:10,000 to 1,000:1, with a preferred range of 1:1,000 to 100:1, and a most preferred range of 1:200 to 10:1.

### A) Nucleic Acid Vectors Useful for Non-Viral Delivery

The invention contemplates the use of a vector containing the gene of interest (i.e., the gene encoding an antigen for which an immune response is desired). The vector may be carried in a delivery vehicle which is targeted or untargeted for cell delivery, as described above. Vectors useful according to the invention will include vectors that integrate into host cell nuclear DNA or stable episomal vectors.

### Episomal Vectors

Extrachromosomal replicators, generally, in addition to their origin function, encode functions that assure equal distribution of replicated molecules between daughter cells at cell division. In higher organisms, different mechanisms exist for partitioning of extrachromosomal replicators. For example, artificial (ARS-containing) plasmids in yeast utilize chromosomal centromeres as extrachromosomal replicators (Struhl et al., 1979, *Proc. Natl. Acad. Sci. USA*, 76:1035-1039). In metazoan cells, one well studied example of a stable extrachromosomal replicator exists - the latent origin oriP from Epstein-Barr Virus (EBV). The maintenance function of EBV requires the viral replication factor EBNA-1 and a series of binding sites for EBNA-1 termed the family of repeats (FR). Replication from oriP requires cis-acting elements (the Family of Repeats - FR and the dyad symmetry element) and the viral origin-binding protein, EBNA-1 (Yates et al., *Proc. Natl. Acad. Sci. USA*, 81, 3806-3810 (1984); Yates et al., *Nature* 313:812-815 (1985)). FR has an effect on the stable extrachromosomal replication of the oriP by nuclear retention of the FR containing plasmids in mitosis. This activity directs plasmids into the newly forming nucleus in the telophase stage of the cell division (Krysan et al., *Mol. Cell. Biol*. 9:1026-1033 (1989)).

Particularly preferred vectors useful according to the invention are maintained at a high copy number in dividing and non-dividing cells of a patient. This may be achieved by employing an episomal vector such as the BPV-I vector system described in WO 94/12629 and in Piirsoo et al., 1996, *EMBO Jour.* 15:1, comprising a plasmid harboring the BPV-1 origin of replication (minimal origin plus minichromosomal maintenance element) and optionally the E1 and E2 genes. The BPV-1 E1 and E2 genes are required for stable maintenance of a BPV episomal vector. These factors ensure that the plasmid is replicated to a stable copy number of up to thirty copies per cell independent of cell cycle status. The gene construct therefore persists stably in both dividing and non-dividing cells. This allows the maintenance of the gene construct in cells such as hemopoietic stem cells and more committed precursor cells.

"Minimal origin" of replication" (MO) refers to a minimal cis-sequence within a papillomavirus that is necessary for initiation of DNA synthesis. The MO of BPV-1 is located at the 3' end of the upstream regulatory region within a 60 base pair DNA fragment (7914-7927) including an AT-rich region, a consensus sequence to which all papilloma viral E2 proteins bind, and an E1 protein binding site spanning nucleotide 1. The MO of HPV is located in the URR fragment (nt 7072-7933/1-99) (Chiang et al. *Proc. Natl. Acad. Sci. USA* 1992).

"E1" refers to the protein encoded by nt 849-2663 of BPV subtype 1; or to nt 832-2779 of HPV of subtype 11, or to equivalent E1 proteins of other papillomaviruses, or to functional fragments or mutants of a papillomavirus E1 protein, i.e., fragments or mutants of E1 which possess the replicating properties of E1.

"E2" refers to the protein encoded by nt 2594-3837 of BPV subtype 1; or to nt 2723-3823 of HPV subtype 11, or to equivalent E2 proteins of other papillomaviruses, or to functional fragments or mutants of a papillomavirus E2 protein, i.e., fragments or mutants of E2 which possess the replicating properties of E2.

"Minichromosomal maintenance element" (MME) refers to a region of the papilloma viral genome to which viral or human proteins essential for papilloma viral replication bind, which region is essential for stable episomal maintenance of the papilloma viral MO in a host cell, as described in Piirsoo et al. Preferably, the MME is a sequence containing multiple binding sites for the transcriptional activator E2. The MME in BPV is herein defined as the region of BPV located within the upstream regulatory region which includes a minimum of about six sequential E2 binding sites, and which gives optimum stable maintenance with about ten sequential E2 binding sites. E2 binding site 9 is a preferred sequence for this site, as described hereinbelow, wherein the sequential sites are separated by a spacer of about 4-10 nucleotides, and optimally 6 nucleotides. E1 and E2 can be provided to the plasmid either in cis or in trans, also as described in WO 94/12629 and in Piirsoo et al.

"E2 binding site" refers to the minimum sequence of papillomavirus double-stranded DNA to which the E2 protein binds. An E2 binding site may include the sequence 5' ACCGTTGCCGGT 3', which is high affinity E2 binding site 9 of the BPV-1 URR; alternatively, an E2 binding site may include permutations of binding site 9, which permutations are found within the URR, and which consist essentially of the consensus sequence 5'ACCN6GGT3' where N is, independent of its position, any nucleotide, and 6 refers to six independent nucleotides (N). One or more transcriptional activator E2 binding sites are, in most papillomaviruses, located in the upstream regulatory region, as in BPV and HPV.

A vector useful according to the invention may include a region of BPV between 6959 - 7945/1 - 470 on the BPV genetic map (see WO 94/12629), which region includes an origin of replication, a first promoter operatively associated with a gene encoding an antigen or epitope thereof, the BPV E1 gene operatively associated with a second promoter to drive transcription of the E1 gene; and the BPV E2 gene operatively associated with a third promoter to drive transcription of the E2 gene.

The promoters which drive expression of the E1 and E2 genes may be identical or different, and may be a tissue-specific promoter, such as the immunoglobulin heavy chain promoter/enhancer for B-cell and the Ig heavy or light chain promoters for blood cell expression, or from ubiquitously expressed genes, for example from the phosphoglycerolkinase, IE-CMV, RSV-LTR or DHFR genes. The arrangement of E1 and E2 genes relative to the BPV origin of replication may mimic the natural orientations of the sequences in the BPV genome, or it may assume a variety of other orientations, the choices of which will be apparent to one of skill in the art.

One skilled in the art will recognize that a variety of vectors will work according to the invention.

### 2. Delivery to APCs Mediated by Viral Vectors

In another preferred approach for introducing nucleic acid and an antigen into an antigen presenting cell, a viral vector containing the nucleic acid is used for transfer. Infection of cells with a viral vector has the advantage that a large proportion of cells receive the nucleic acid. Additionally, molecules encoded within the viral vector are expressed efficiently in cells which have taken up the vector nucleic acid.
1. Retroviruses: Defective retroviruses are well characterized for use in gene transfer for gene therapy purposes (for a review see Miller, A.D. (1990) *Blood* 76:271). A recombinant retrovirus can be constructed having a nucleic acid encoding an antigen of interest inserted into the retroviral genome. Additionally, portions of the retroviral genome can be removed to render the retrovirus replication defective. The replication defective retrovirus is then packaged into virions which can be used to infect a target cell through the use of a helper virus by standard techniques. Protocols for producing recombinant retroviruses and for infecting cells *in vitro* or *in vivo* with such viruses can be found in Current Protocols in Molecular Biology, Ausubel, F.M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include ψCrip, ψCre, ψ2, and ψAm. Retroviruses have been used to introduce a variety of genes into many different cell types, including epithelial cells, endothelial cells, lymphocytes, myoblasts, hepatocytes, bone marrow cells, *in vitro* and/or *in vivo* (see for example Eglitis, et al. (1985) *Science* 230:1395-1398; Danos and Mulligan (1988) *Proc. Natl.* Acad. *Sci. USA* 85:6460-6464; Wilson et al. (1988) *Proc. Natl. Acad. Sci. USA* 85:3014-3018; Armentano et al. (1990) *Proc. Natl. Acad. Sci. USA* 87:6141-6145; Huber et al. (1991) *Proc. Natl. Acad. Sci. USA* 88:8039-8043; Ferry et al. (1991) *Proc. Natl. Acad Sci. USA* 88:8377-8381; Chowdhury et al. (1991) *Science* 254:1802-1805; van Beusechem et al. (1992) *Proc. Natl. Acad Sci. USA* 89:7640-7644; Kay et al. (1992) Human *Gene Therapy* 3:641-647; Dai et al. (1992) *Proc. Natl. Acad Sci. USA* 89:10892-10895; Hwu et al. (1993) *J. Immunol.* 150:4:104-115; U.S. Patent No. 4,868,116; U.S. Patent No. 4,980,286; PCT Application WO 89/07136; PCT Application WO 89/02468; PCT Application WO 89/05345; and PCT Application WO 92/07573).
2. Adenoviruses: The genome of an adenovirus can be manipulated such that it encodes and expresses a gene product of interest but is inactivated in terms of its ability to replicate in a normal lytic viral life cycle. See for example Berkner et al. (1988) *BioTechniques* 6:616; Rosenfeld et al. (1991) *Science* 252:431-434; and Rosenfeld et al. (1992) *Cell* 68:143-155. Suitable adenoviral vectors derived from the adenovirus strain Ad type 5 d1324 or other strains of adenovirus (e.g., Adz, Ad3, Ad7 etc.) are well known to those skilled in the art. Recombinant adenoviruses are advantageous in that they do not require dividing cells to be effective gene delivery vehicles and can be used to infect a wide variety of cell types, including airway epithelium (Rosenfeld et al. (1992) cited *supra*), endothelial cells (Lemarchand et al. (1992) *Proc. Natl. Acad. Sci. USA* 89:6482-6486), hepatocytes (Herz and Gerard (1993) *Proc. Natl. Acad. Sci. USA* 90:2812-2816) and muscle cells (Quantin et al. (1992) *Proc. Natl. Acad. Sci. USA* 89:2581-2584). Many replication-defective adenoviral vectors are deleted for all or parts of the viral E1 and E3 genes but retain as much as 80 % of the adenoviral genetic material.
3. Adeno-Associated Viruses: Adeno-associated virus (AAV) is a naturally occurring defective virus that requires another virus, such as an adenovirus or a herpes virus, as a helper virus for efficient replication and a productive life cycle. (For a review see Muzyczka et al. *Curr. Topics in Micro*. *and Immunol*. (1992) 158:97-129). It exhibits a high frequency of stable integration (see for example Flotte et al. (1992) *Am. J. Respir. Cell. Mol. Biol.* 7:349-356; Samulski et al. (1989) J. Virol. 63:3822-3828; and McLaughlin et al. (1989) *J. Virol* 62:1963-1973). Vectors containing as little as 300 base pairs of AAV can be packaged and can integrate. Space for exogenous nucleic acid is limited to about 4.5 kb. An AAV vector such as that described in Tratschin et al. (1985) *Mol. Cell. Biol.* 5:3251-3260 can be used to introduce nucleic acid into cells. A variety of nucleic acids have been introduced into different cell types using AAV vectors (see for example Hermonat et al. (1984) *Proc. Natl. Acad. Sci. USA* 81 :6466-6470; Tratschin et al. (1985) *Mol. Cell. Biol.* 4:2072-2081; Wondisford et al. (1988) *Mol. Bndocrinol.* 2:32-39; Tratschin et al. (1984) *J. Virol*.51:611 - 619; and Flotte et al. (1993) *J. Biol. Chem.* 268:3781-3790).

The delivery vehicle also may comprise a viral antigen such as any of those described above, wherein one or more of the viral proteins has been modified to include an epitope(s) to which an immune response is desired.

### 3. Tissue-Restricted Gene Expression

Alternatively or in addition to the use of targeted delivery vehicles, DNA regulatory elements which lead to expression in APCs, their Stem cells or other precursor cell types can be used according to the invention.

Tissue specific expression is provided according to the invention using genetic control elements which restrict expression of the gene with which the element is associated to a tissue for which the element is specific. Examples of such genetic control elements include locus control regions and tissue-specific promoters and enhancers.

Locus Control Regions (LCRs) (Grosveld et al., *Cell* 51:975-985, 1987), also known as Dominant Activator Sequences, Locus Activating Regions or Dominant Control Regions, are responsible for conferring tissue specific, integration-site independent, copy number dependent expression on transgenes integrated into chromatin in host cells. First discovered in the human globin gene system, which was prone to strong position effects when integrated into the chromatin of transgenic mice or mouse erythroleukaemia (MEL) cells (Magram et al., *Nature* 315:338-340, 1985; Townes et al., *EMBO J.* 4:1715-1723, 1985; Kollias et al., *Cell* 46:89-94, 1986; Antoniou et al., *EMBO J.* 7:377-384, 1988), LCRs have the ability to overcome such position effects when linked directly to transgenes (Grosveld et al., supra). Numerous LCRs have been defined in the art, including but not limited to the β-globin and CD2 LCRs (Greaves et al., 1989), the macrophage-specific lysozyme LCR (Bonifer et al., 1985, 1990), and a class II MHC LCR (Carson et al., *Nucleic Acids Res.* 21, 9:2065-2072, 1993).

Preferred regulatory elements include locus control regions (LCRs) such as the MHC class II LCR. Other control regions, for example, the Ig LCR for B cells, may also be used provided they lead to expression in APCs, their Stem cells or other precursor cell types.

### Vectors encoding elements permitting immune system evasion.

Vectors useful according to the invention also may encode a sequence which serves to protect certain antigenic determinants encoded by the vector from viability to the host immune system. Thus, if the host mounts an immune response to determinants encoded by the vector, or carried on a vehicle used to deliver the nucleic acid to the antigen presenting cell, then this antigenicity may be eliminated or reduced by introducing into the open reading frame of the antigenic protein sequences from the Epstein bar virus EBNA-1 protein encoding a Glycine-containing repeat, that suppresses antigen presentation of amino acid sequences linked in cis. See for example, Levitskaya et al., 1995, *Nature* 375:685.

A DNA sequence encoding a Glycine-containing sequence (for example, residues 93-325 of the Epstein-Barr virus nuclear antigen-1, carried on an Nco1-Apa1 fragment of EBNA1 (strain B95.8)) is inserted into a selected site within a gene encoding a protein which is believed or known to be immunogenic, but for which no immune response is desired upon administration of a nucleic acid according to the invention. The Glycine-encoding DNA sequence may, for example, be inserted downstream of the protein which is desired to be non-immunogenic, or downstream of an epitope of the protein which is known to be immunogenic; for example, it may be joined to the protein via an in-frame fusion at the carboxy or amino terminus of the protein, or inserted anywhere within the protein to render that protein non-immunogenic. It is preferred that the glycine-containing sequence is inserted within the protein at a distance wherein either terminal residue of the glycine-containing sequence is at a distance of between about 1 and 300 residues from an immunogenic epitope of the protein. In the case of a protein which contains multiple epitopes, it is believed that all epitopes of the antigenic protein will be rendered non-antigenic according to this embodiment of the invention. Preferably, the distance between the site of insertion in the protein of the glycine-containing sequence and one amino acid of the protein epitope is between about 1 and 200 residues, more preferably 10-100 residues, and most preferably between about 20 and 50 residues. As used herein, "epitope" refers to an antigenic region of about 6-20 amino acids within a protein that is immunologically recognized as foreign.

For example, where the vector used to deliver a nucleic acid encoding an antigen to antigen presenting cells according to the invention contains the BPV E1 and E2 proteins, it is possible that the E1 and E2 proteins may cause an undesired immune response. Therefore, insertion of a glycine-containing sequence into each reading frame of these proteins may be accomplished in order to reduce or eliminate an immune response to the E1 and E2 proteins, without appreciably affecting the desired immune response to the antigen encoded by the vaccinating nucleic acid.

Glycine-containing sequence useful for obtaining masking of selected foreign proteins or immunogenic epitopes, such as those carried in vectors useful according to the invention, comprise a recombinant glycine-containing amino acid sequence having the formula: [(Glyₐ)X(Gly_{b})Y(Gly_{c})Z]n, wherein each Glyₐ, Gly_{b}, Gly_{c}, independently, may be one, two, or three sequential glycine residues; each of X, Y and Z is, independently, a hydrophobic or polar amino acid without a ring structure and having a side-chain of less than 3 atoms, wherein each of X, Y and Z, respectively, need not be identical from n repeat to n repeat; and n may be from 1-66.

It is preferred that each of X, Y and Z is, independently, selected from the group consisting of Ala, Ser, Val, Ile, Leu and Thr; more preferably, each of X, Y and Z is, independently, one of Ala and Ser; most preferably, each of X, Y and Z is, independently, Ala; more preferably, each of X and Y is not Met or Cys.

It is also preferred that the glycine-containing sequence comprises (GlyGlyXGlyYGlyZ) and n=7 repeats; or (GlyGlyXGlyYGlyGlyZ) in n=9 repeats where the remaining n repeats comprise (Glyₐ)X(Gly_{b})Y(Gly_{c})Z up to a total repeat number of 66, and preferably 28 total repeats; or the glycine-containing sequence is (GlyGlyXGlyYGlyGlyGlyZ) in n=7 repeats where the remaining n repeats comprise (Glyₐ)X(Gly_{b})Y(Gly_{c})Z up to a total repeat number of 66, and preferably 28 total repeats; or the glycine-containing sequence comprises (GlyGlyAlaGlyAlaGlyGlyAla) in n=9 repeats where the remaining n repeats comprise (Glyₐ)X(Gly_{b})Y(Gly_{c})Z up to a total repeat number of 66, and preferably 28 total repeats.

It is also preferred that the glycine-containing sequence is (GlyGlyAlaGlyAlaGlyGlyGlyAla) in n=7 repeats where the remaining n repeats comprise (Glyₐ)X(Gly_{b})Y(Gly_{c})Z up to a total repeat number of 66, and preferably 28 total repeats; or the glycine-containing sequence is (GlyGlyXGlyYGlyGlyZ) in n=9 repeats and (GlyGlyXGlyYGlyGlyGlyZ) in n=7 repeats where the remaining n repeats comprise (Glyₐ)X(Gly_{b})Y(Gly_{c})Z up to a total repeat number of 66.

The glycine-containing sequence (GlyGlyAlaGlyAlaGlyGlyAla) in n=9 repeats and (GlyGlyAlaGlyAlaGlyGlyGlyAla) in n=7 repeats is also preferred, where the remaining n repeats comprise (Glyₐ)X(Gly_{b})Y(Gly_{c})Z up to a total repeat number of 66.

### Antigens and Genes Encoding Antigens

### Useful According to the Invention

It is contemplated according to the invention that an immune response may be elicited via presentation of any protein or peptide capable of eliciting such a response. It is preferred that the antigen is a key epitope which gives rise to a strong immune response to a particular agent of infectious disease. If desired, more than one antigen or epitope may be encoded by the nucleic acid in order to increase the likelihood of an immune response. Thus, the vector may include a multi-gene construct in which the antigens encoded by the genes are coordinately expressed. If desired, all of the open reading frames of a pathogen's genome may be arranged together (as an expression library) in a vector in order to form a region of coding sequences which are expressed and presented by the antigen presenting cell. The use of expression libraries is described in Barry et al., 1995, Nature 377:632.

Alternatively, DNA sequences encoding antigens specific for different diseases or infectious agents may be arranged together in a vector for delivery to and expression in an antigen presenting cell. Therefore, immune protection may be elicited against a number of diseases specified by the antigens encoded within the construct.

In a further embodiment of the present invention the antigen encoding nucleic acid sequence may contain a signal sequence for secretion of the antigen outside the cell. Signal sequences useful in the present invention are well known to those skilled in the art and are, for example, described in Blobel and Dobberstein (1975), *J. Cell Biol.,* 67, 852-862. The secreted antigen will be taken up by the secreting cell and other neighbouring cells, processed as an exogenous antigen, and presented in association with Class II MHC. The delivery vehicle of the present invention may contain a mixture of nucleic acids encoding an antigen, some with, and some without, the secretion signal sequence. Thus secreted and non-secreted antigen can be produced in the same cell and both Class I and Class II NHC presentation can be produced after a single transfection event.

Antigens Useful According to the Invention are as follows.
1. Viral Antigens
   Examples of viral antigens include, but are not limited to, retroviral antigens such as retroviral antigens from the human immunodeficiency virus (HIV) antigens such as gene products of the *gag, pol*, and *env* genes, the Nef protein, reverse transcriptase, and other HIV components; hepatitis viral antigens such as the S, M, and L proteins of hepatitis B virus, the pre-S antigen of hepatitis B virus, and other hepatitis, e.g., hepatitis A, B, and C, viral components such as hepatitis C viral RNA; influenza viral antigens such as hemagglutinin and neuraminidase and other influenza viral components; measles viral antigens such as the measles virus fusion protein and other measles virus components; rubella viral antigens such as proteins E1 and E2 and other rubella virus components; rotaviral antigens such as VP7sc and other rotaviral components; cytomegaloviral antigens such as envelope glycoprotein B and other cytomegaloviral antigen components; respiratory syncytial viral antigens such as the RSV fusion protein, the M2 protein and other respiratory syncytial viral antigen components; herpes simplex viral antigens such as immediate early proteins, glycoprotein D, and other herpes simplex viral antigen components; varicella zoster viral antigens such as gpI, gpII, and other varicella zoster viral antigen components; Japanese encephalitis viral antigens such as proteins E, M-E, M-E-NS 1, NS 1, NS 1 -NS2A, 80%E, and other Japanese encephalitis viral antigen components; rabies viral antigens such as rabies glycoprotein, rabies nucleoprotein and other rabies viral antigen components. See Fundamental Virology, Second Edition, e's. Fields, B.N. and Knipe, D.M. (Raven Press, New York, 1991) for additional examples of viral antigens.
2. Bacterial antigens
   Bacterial antigens which can be used in the compositions and methods of the invention include, but are not limited to, pertussis bacterial antigens such as pertussis toxin, filamentous hemagglutinin, pertactin, FIM2, FIM3, adenylate cyclase and other pertussis bacterial antigen components; diptheria bacterial antigens such as diptheria toxin or toxoid and other diptheria bacterial antigen components; tetanus bacterial antigens such as tetanus toxin or toxoid and other tetanus bacterial antigen components; streptococcal bacterial antigens such as M proteins and other streptococcal bacterial antigen components; grarn- negative bacilli bacterial antigens such as lipopolysaccharides and other gram-negative bacterial antigen components; Mycobacterium tuberculosis bacterial antigens such as mycolic acid, heat shock protein 65 (HSP65), the 30kDa major secreted protein, antigen 85A and other mycobacterial antigen components; Helicobacter pylori bacterial antigen components; pneumococcal bacterial antigens such as pneumolysin, pneumococcal capsular polysaccharides and other pneumococcal bacterial antigen components; haemophilus influenza bacterial antigens such as capsular polysaccharides and other haemophilus influenza bacterial antigen components; anthrax bacterial antigens such as anthrax protective antigen and other anthrax bacterial antigen components; rickettsiae bacterial antigens such as romps and other rickettsiae bacterial antigen components. Also included with the bacterial antigens described herein are any other bacterial, mycobacterial, mycoplasmal, rickettsial, or chlamydial antigens.
3. Fungal antigens
   Fungal antigens which can be used in the compositions and methods of the invention include, but are not limited to, candida fungal antigen components; histoplasma fungal antigens such as heat shock protein 60 (HSP60) and other histoplasma fungal antigen components; cryptococcal fungal antigens such as capsular polysaccharides and other cryptococcal fungal antigen components; coccidiodes fungal antigens such as spherule antigens and other coccidiodes fungal antigen components; and tinea fungal antigens such as trichophytin and other coccidiodes fungal antigen components.
4. Parasite antigens
   Examples of protozoa and other parasitic antigens include, but are not limited to, plasmodium falciparum antigens such as merozoite surface antigens, sporozoite surface antigens, circumsporozoite antigens, gametocyte/gamete surface antigens, blood-stage antigen pf 1 55/RESA and other plasmodial antigen components; toxoplasma antigens such as SAG-1, p30 and other toxoplasma antigen components; schistosomae antigens such as glutathione-S-transferase, paramyosin, and other schistosomal antigen components; leishmania major and other leishmaniae antigens such as gp63, lipophosphoglycan and its associated protein and other leishmanial antigen components; and trypanosoma cruzi antigens such as the 75-77kDa antigen, the 56kDa antigen and other trypanosomal antigen components.
5. Tumor antigens
   Tumor antigens which can be used in the compositions and methods of the invention include, but are not limited to, prostate specific antigen (PSA), telomerase; multidrug resistance proteins such as P-glycoprotein; MAGE-1, alpha fetoprotein, carcinoembryonic antigen, mutant p53, papillomavirus antigens, gangliosides or other carbohydrate- containing components of melanoma or other tumor cells. It is contemplated by the invention that antigens from any type of tumor cell can be used in the compositions and methods described herein.
6. Antigens involved in autoimmunity
   Antigens which have been shown to be involved in autoimmunity and could be used in the delivery vehicles of the present invention to induce tolerance include, but are not limited to, myelin basic protein, myelin oligodendrocyte glycoprotein and proteolipid protein of multiple sclerosis and CII collagen protein of rheumatoid arthritis.
   It is known that bacterial DNA can have immunostimulatory properties (Tokunaga et al., J. Nat. Cancer Insti., 1984, 72:955; Togunaga et al., Microbiol. Immunol., 1992, 36:55). Thus, for a nucleic acid encoding any one or more of the above-described antigens, it is preferred that the nucleic acid has immunostimulatory properties and may contain specifically identified CpG motifs that have been demonstrated to confer immunostimulation (Krieg et al., Nature, 1995, 374:546; Yi et al., Jour. Immunol., 1996, 156:558).

### DOSAGE, ROUTE OF ADMINISTRATION AND PHARMACEUTICAL FORMULATIONS

According to the present invention, nucleic acid encoding one or more antigens or epitopes thereof may be used in gene therapy in order to provide immune protection against various infectious agents and diseases. The nucleic acid may be delivered to antigen presenting cells as part of a vehicle for delivering a nucleic acid to a cell which may specifically target a cell type or which may be untargeted with respect to the recipient cell. Delivery of a nucleic acid to APCs according to the present invention may be accomplished via *ex vivo* gene therapy or in *in vivo* gene therapy. For prophylactic and therapeutic vaccines, in vivo gene therapy is preferred, and for therapeutic vaccines, ex vivo gene therapy also is appropriate. A gene may be delivered to cells cultured *ex vivo* prior to reinfusion of the transfected cells into the patient or the gene may be delivered in a gene delivery vehicle complex by direct *in vivo* injection into the patient's vascular system or in a body area rich in the target cells. The *in vivo* injection may be made subcutaneously, intravenously, intramuscularly or intraperitoneally. The delivery vehicle may also be delivered by oral, nasal, vaginal or urethral routes, and may be delivered in combination with other delivery vehicles such as microspheres, gene gun, or Bioject™. Techniques for *ex vivo* and *in vivo* gene therapy are known to those skilled in the art. Generally, the compositions are administered in a manner compatible with the dosage formulation, and in such amount as will be prophylactically and/or therapeutically effective. The quantity to be administered depends on the subject to be treated, including, e.g., capacity of the subject's immune system to synthesize antibodies, and the degree of protection desired. Suitable dosage ranges are on the order of, where *ex vivo* transfected cells are administered to a patient 10⁵ - 10⁸, and optionally 10⁶ - 10⁷ cells are administered in a single dose; where a gene/delivery vehicle complex is administered 100ng - 10mg, or 1ug - 1mg, or optionally lug - 10 ug of complex or mixture is administered in a single dose. Suitable regimens for initial administration and booster shots are also variable but are typified by an initial administration followed by subsequent inoculations or other administrations. Precise amounts of active ingredient required to be administered depend on the judgment of the practitioner and may be peculiar to each subject. It will be apparent to those of skill in the art that the therapeutically effective amount of a composition of this invention will depend, *inter alia*, upon the administration schedule, the unit dose of antigen administered or expressed by an encoding nucleic acid that is administered, whether the compositions are administered in combination with other therapeutic agents, the immune status and health of the recipient, and the therapeutic activity of the particular nucleic acid molecule, delivery complex, or *ex vivo* transfected cell.

Complexes and mixtures according to the invention also may be mixed in a physiologically acceptable diluent such as water, phosphate buffered saline, or saline, and further may include an adjuvant. Adjuvants such as incomplete Freund's adjuvant, aluminum phosphate, aluminum hydroxide, or alum are materials well known in the art. Upon administration with a composition as described herein, via injection, oral, transdermal, or other routes, the immune system of the host will respond to the polypeptide antigen or the nucleic acid encoded antigen and the polypeptide antigen by producing either an effective cellular or humoral immune response to the antigen(s) or both effective cellular and humoral immune respones, as described herein.

Compositions of the invention can be given in a single dose schedule, or in a multiple dose schedule. A multiple dose schedule is one in which a primary course of vaccination can include 1-10 separate doses, followed by other doses given at subsequent time intervals required to maintain and or reinforce the immune response, for example, at 1-4 months for a second dose, and if needed, a subsequent dose(s) after several months. Periodic boosters at intervals of 1-5 years, usually 3 years, may be desirable to maintain the desired levels of protective immunity. The course of the immunization can be followed by *in vitro* proliferation assays of peripheral blood lymphocytes (PBLs) co-cultured with ESAT6 or ST- CF, and by measuring the levels of IFN-γ released from the primed lymphocytes. The assays can be performed using conventional labels, such as radionucleotides, enzymes, fluorescent labels and the like. These techniques are known to one skilled in the art and can be found in US Patent Nos. 3,791,932, 4,174,384 and 3,949,064.

In addition to allowing maximization of the immune response the ability to co-deliver DNA and antigen to APCs has the advantage of being able to modulate the type of immune response induced. It is known that DNA immunization tends to produce a T helper-type 1 response (Th1) whereas protein immunization induces a T helper-type 2 (Th2) response (Raz et al., 1996, PNAS 93:5141). Thus, by manipulation of the DNA and protein/peptide components of the complex or mixture of the invention, the type of immune response may be manipulated by varying the relative amounts of each component.

Complexes and mixtures of the invention also may find use as diagnostic reagents. For example, they may be used to determine the susceptibility of a particular individual to a treatment regimen which employs the complexes or mixtures, and thus may be helpful in modifying an existing treatment protocol or in determining a prognosis for an affected individual. In addition, they may be used to predict which individuals will be at substantial risk for developing an infection by an organism, pathogen or agent for which vaccination is desired.

The invention may be used to generate both prophylactic and therapeutic immune responses. Optionally, the immune system can be primed and boosted either using the same complex or mixture or using combinations of different complexes or mixtures described herein, or combinations of complexes or mixtures described herein with conventional vaccines known in the art, e.g., recombinant protein vaccines or killed virus vaccines.

### EXEMPLIFICATION

### 1. The following examples demonstrate cell type-specific expression in antigen presenting cells in animal models.

### Cell type-specific expression of the human glucocerebrosidase gene in cells of the monocyte-macrophage lineage in several lines of transgenic mice.

A construct was generated in which the mouse MHC class II Ea gene Locus Control Region (LCR, Carson and Wiles, Nucl. Acids Res., 21, (2065-2072 (1993)) was linked to the human gene encoding glucocerebrosidase (GC). Several transgenic lines were made with linearized DNA of this construct. Expression of human GC and functional activity was detectable in the spleen cells of most transgenic lines. Non-transgenic mice (ntg) had no detectable human GC activity. The spleen contains both B-lymphocytes and macrophages/dendritic cells, and so to definitively demonstrate expression in macrophages/dendritic cells, enriched populations of activated, transgenic macrophages were produced by thioglycollate injection into the peritoneum of transgenic mice. Analysis of the resulting macrophage populations for expression of human GC showed that F4/80-positive macrophages (Gordon et al., *Curr. Topics. Microbiol. Immunol*., (1992) 181, 1-37) abundantly expressed human GC (as detected by monoclonal antibody 8E4). This demonstrates APC-specific expression of the heterologous gene using the MHC LCR.

### In vivo Vaccination: generation of transgenic mice expressing Influenza NP under MHC LCR control, and generation of anti-NP immunity in syngeneic non-transgenic littermates by transplantation of transgenic spleen and bone marrow cells.

To demonstrate that expression of foreign proteins in the APC can generate protective cellular and/or humoral immune responses, transgenic lines are made in which the Influenza NP gene is expressed under the control of the MHC class II Ea LCR as described above.

From these mice, spleen cells or bone marrow cells are isolated and infused into non-transgenic syngeneic mice. Mice are then analyzed for expression of the NP gene, which is expected to be present in the donor cells and their differentiated descendants. Analysis of the transplant recipients is expected to reveal that some have successfully generated NP-specific cytotoxic T cells and anti-NP antibody responses.

### Anti-Influenza vaccines to generate immunity directed against the Hemagglutinin component of the viral capsid.

Transgenic lines are made in which the Influenza Hemagglutinin (HA) gene is expressed under the control of the MHC class II Ea LCR described above. From these mice, spleen cells or bone marrow cells are isolated and infused into non-transgenic syngeneic mice. Mice are then analyzed for expression of the HA gene, which is shown to be present in the donor cells and their differentiated descendants. Analysis of the transplant recipients is expected to reveal that some have successfully generated HA-specific cytotoxic T cells and anti-HA antibody responses.

### Anti-HIV vaccines

Transgenic mice are made expressing HIV-1 tat, rev, nef or gag genes, and combinations thereof, under the control of the MHC class II Ea LCR described above. Transplantation of transgenic spleen cells to syngeneic non-transgenic mice is expected to elicit a cytotoxic T cell response which can be measure *in vitro* using Chromium⁵¹ release assays for T cell-mediated cytotoxicity. To measure the T cell response, T cells from transplantation recipients are challenged with syngeneic, non-transgenic, irradiated, Cr⁵¹-labeled target Antigen-Presenting Cells in the presence of immunodominant peptides from the HIV-1 nef, rev, gag and tat proteins. Effective T cell responses are detected by measuring Cr⁵¹ release as a result of target cell lysis. Serum antibody responses against HIV-1 nef, rev, gag and tat are also measured in the transplant recipients, to confirm the generation of a humoral response.

### Anti-Hepatitis B virus (HBV) vaccines

Transgenic mice are made expressing the HBc, HBe, S, pre-S and pX genes, and combinations thereof, under the control of the MHC class II Ea LCR. Transplantation of transgenic spleen cells to syngeneic non-transgenic mice is expected to elicit a cytotoxic T cell response which can be measured *in vitro* using Chromium⁵¹ release assays for T cell-mediated cytotoxicity. To measure the T cell response, T cells from transplantation recipients are challenged with syngeneic, non-transgenic, irradiated, Cr⁵¹-labeled target Antigen-Presenting Cells in the presence of immunodominant peptides from the HBc, HBe, S, pre-S, and pX proteins. Effective T cell responses are detected by measuring Cr⁵¹ release as a result of target cell lysis. Serum antibody responses against the HBc, HBe. S, pre-S, and pX proteins are also measured in the transplant recipients, to confirm the generation of a humoral response.

### Anti-Hepatitis C virus (HCV) vaccines

Transgenic mice are made expressing nucleocapsid protein C22-3, the NS3 and NS4-region derived C200 and C33c proteins, and combinations thereof, under the control of the MHC class II Ea LCR. Transplantation of transgenic spleen cells to syngeneic non-transgenic mice is expected to elicit a cytotoxic T cell response which can be measured *in vitro* using Chromium⁵¹ release assays for T cell-mediated cytotoxicity. To measure the T cell response, T cells from transplantation recipients are challenged with syngeneic, non-transgenic, irradiated, Cr⁵¹-labeled target Antigen-Presenting Cells in the presence of immunodominant peptides from nucleocapsid protein C22-3, the NS3 and NS4- region derived C200 and C33c proteins. Effective T cell responses are detected by measuring Cr⁵¹ release as a result of the target cell lysis. Serum antibody responses against the nucleocapsid protein C22-3, the NS3 and NS4- region derived C200 and C22c proteins are also measured in the transplant recipients, to confirm the generation of a humoral response.

### Anti-Human Papilloma Virus (HPV) vaccines

Transgenic mice are made expressing the HPV 16 proteins E1, E2, E7, E5 and E6, and combinations thereof, under the control of the MHC class II Ea LCR. Transplantation of transgenic spleen cells to syngeneic non-transgenic mice is expected to elicit a cytotoxic T cell response which can be measured *in vitro* using Chromium⁵¹ release assays for T cell-mediated cytotoxicity. To measure the T cell response, T cells from transplantation recipients are challenged with syngeneic, non-transgenic, irradiated, Cr⁵¹-labeled target Antigen-Presenting Cells in the presence of immunodominant peptides from the E1, E2, E7, E5 and E6 proteins. Effective T cell responses are detected by measuring Cr⁵¹ release as a result of target cell lysis. Serum antibody responses against the E1, E2, E7 E5 and E6 proteins are also measured in the transplant recipients, to confirm the generation of a humoral response.

### Anti-tumor vaccines

Lines of transgenic mice are made expressing the genes encoding the melanoma-specific antigen MAGE-1, tyrosinase, the murine homologue of the HER2/neu proto-oncogene which is mutated in ovarian and breast tumors, the HPV 16 E7 protein, and the connexin 37 protein which is mutated in the 3LL lung carcinoma. Bone marrow and spleen cells of these transgenic mice are transplanted to syngeneic non-transgenic mice to elicit cytotoxic T-cell mediated and humoral immune response to the proteins encoded by the transgenes. MAGE-1/tyrosinase, HER2/neu, HPV-16 E7, and connexin 37 transgenic-transplant recipients and untransplanted controls are subsequently injected subcutaneously with tumor cells from the B16 mouse melanoma, murine mammary carcinoma T50/80, the 3LL mouse lung carcinoma, or the C3 mouse tumor transfected with HPV 16 genomic DNA, respectively, and tumor growth is monitored. Transplant recipients are expected to show much reduced tumor growth than non-transplanted controls, confirming that protective anti-tumorimmune responses have been generated.

Moreover, non-transplanted recipients already bearing tumors of the types described above are expected to be cured of their cancers by an infusion of bone marrow or spleen cells from syngeneic mice transgenic for the corresponding tumor-specific antigen-coding gene also described above.

### 2. Examples of gene therapy using the delivery vehicle of the present invention.

### Anti-Influenza vaccines to generate impunity directed against the Hemagglutinin component of the viral capsid

A delivery vehicle comprising a plasmid vector in which the Influenza Hemagglutinin (HA) gene is expressed under the control of the MHC class II Ea LCR described above, is administered to subjects by direct intravascular injection or by transfection of cultured bone marrow-derived cells *in vitro* which are then reinfused into patients intravenously. Subsequent analysis of the patients is expected to reveal that some patients have successfully generated HA-specific cytotoxic T cells and anti-HA antibody responses.

A delivery vehicle comprising a plasmid vector in which nucleotide sequences from the influenza matrix protein gene encoding a peptide including the HLA A2 restricted epitope located at residues 57-68 (Goton et al, 1987, Nature 326:331-332. Moss et al 1991, PNAS 88:8987-8990) are expressed under the control of the MHC class II Ea LCR described above is administered to patients by direct intravascular injection or by transfection of *cultured* bone marrow-derived cells *in vitro* which are then reinfused into patients intravenously. Subsequent analysis of the patients is expected to reveal that some patients have successfully generated HA-specific cytotoxic T cells.

### Anti-HIV vaccines

A delivery vehicle comprising a plasmid vector in which the HIV-1 tat, rev, nef or gag genes, and combinations thereof, are expressed under the control of the MHC class II Ea LCR is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against the HIV-1 nef, rev, tat and gag proteins. Effective T cell responses are detected by measuring Cr⁵¹ release as a result of target cell lysis. Serum antibody responses against HIV-1 nef, rev, tat and gag are also measured in the transplant recipients, confirming the generation of humoral response.

A delivery vehicle comprising a plasmid vector containing nucleotide sequences from the HIV-1 gag gene which encode peptides including the HLAB27 and B8 restricted epitopes in HIV p17 Gag and HIV p24 Gag (Nixon et al. 1988, *Nature* 336:484-487, Nixon and McMichael, 1991 *AIDS* 5:1 e049-1059) and combinations thereof, expressed under the control of the mHC class II Ea LCR, is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against Gag protein sequences. Effective T cell responses are detected by measuring Cr⁵¹ release as a result of target cell lysis.

### Anti-Hepatitis B virus (HBV) vaccines

A delivery vehicle comprising a plasmid vector in which HBV HBc, HBe, S, pre-S, and pX genes, and combinations thereof, are expressed under the control of the MHC class II Ea LCR is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against the HBV HBc, HBe, S, pre-S, and pX proteins. Effective T cell responses are detected by measuring Cr⁵¹ release as a result of target cell lysis. Serum antibody responses against HBV HBc, HBe, S, pre-S, and pX proteins are also measured in the transplant recipients, confirming the generation of humoral response.

### Anti-Hepatitis C virus (HCV) vaccines

A delivery vehicle comprising a plasmid vector in which the genes encoding the HCB nucleocapsid protein C22-3, the NS3 and NS4 -region-derived C200 and C33c proteins, and combinations thereof, are expressed under the control of the MHC class II Ea LCR is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against nucleocapsid protein C22-3, the NS3 and NS4 -region-derived C200 and C22c proteins. Effective T cell responses are detected by measuring Cr⁵¹ release as a result of target cell lysis. Serum antibody responses against nucleocapsid protein C22-3, the NW3 and NS4 -region-derived C200 and C33c proteins are also measured in the transplant recipients, confirming the generation of a humoral response.

### Anti-Human Papilloma Virus (HPV) vaccines

A delivery vehicle comprising a plasmid vector in which genes encoding the HPV 16 proteins E1, E2, E7 E5 and E6, and combinations thereof, are expressed under the control of the MHC class II Ea LCR, is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against E1, E2, E7 E5 and E6 proteins. Effective T cell responses are detected by measuring Cr⁵¹ release as a result of target cell lysis. Serum antibody responses are also measured in the transplant recipients, confirming the generation of a humoral response.

### Anti-tumor vaccines

A delivery vehicle comprising a plasmid vector in which genes encoding the melanoma-specific antigen MAGE-1, tyrosinase, the murine homologue of the HER2/neu proto-oncogene which is mutated in ovarian and breast tumors, and the HPV 16 E7 protein are expressed under the control of the MHC class II Ea LCR, is administered to patients by direct intravascular injection or by transfection of cultured bone marrow-derived cells *in vitro* which are then reinfused into patients intravenously. Subsequent analysis of the patients is expected to reveal that some patients have successfully generated CTL responses against MAGE-1, tyrosinase, HER2/neu or HPV E6 or E7 proteins. Effective T cell responses are detected by measuring Cr⁵¹ release as a result of target cell lysis. Serum antibody responses against MAGE-1, tyrosinase, HER2/neu, or HPV E6 or E7 proteins are also measured in the transplant recipients, confirming the generation of humoral response. In addition tumor regression may be observed.

### Examples of delivery systems that allow presentation of antigen in association with Class I and Class II MHC (in vivo).

The following examples teach one of skill in the art how to differentiate MHC Class I and Class II responses *in vivo*, and how to obtain an increased MHC Class II-mediated immune response using a construct prepared and administered according to the invention.

### Addition of peptide/protein to the delivery vehicle

To demonstrate that administration of a delivery system containing peptide/protein antigens gives rise to a greater helper T cell response (i.e. predominantly a class II restricted response) than administration of a delivery vehicle in which the antigen is solely encoded on the nucleic acid, mice are vaccinated with a delivery vehicle with or without antigenic peptides. The complex intended for delivery will have previously been tested for delivery of both antigen and DNA in vivo. Testing is performed by screening complex formulations for the ability to generate an immune response within the animals. Possible complex formulations include, but are not limited to, those described in International Patent Application No. PCT/GB96/01396. The DNA contained within the delivery vehicle either encodes no antigenic gene or the gene for influenza nucleoprotein. The antigenic peptides are appropriate, Class II presented, epitopes of NP or the whole NP protein. T cells from the immunized mice are then assayed for class I- and class II-restricted responses. When the delivery vehicle contained antigenic peptide/protein but no antigen gene a predominantly class II-restricted response is observed, whereas in the absence of both antigenic peptides/protein, and antigen gene, no class II-restricted response is seen. In the presence of the antigen gene, class I and class II responses are observed but the class II response is increased when antigenic peptides/protein are included on the delivery vehicle. Thus, addition of antigenic peptide/protein to the delivery vehicle produces an increased class II-restricted response. Class I-restricted CTL responses are measured by Cr⁵¹ release assay and Class II-restricted helper T cell responses are measured by T cell proliferation assay or cytokine release assay.

### Addition of secretion signal sequence to antigen gene

To demonstrate that administration of a delivery vehicle containing DNA encoding an antigen gene with a secretion signal sequence that is functional in mammalian cells gives rise to a greater Class II response than administration of a delivery vehicle in which an non-secreted version of the antigen is produced, mice are vaccinated with a delivery vehicle containing a DNA molecule encoding the influenza nucleoprotein gene with or without a secretory signal sequence. T cells from the immunized mice are then assayed for CTL and helper T cell responses in order to determine the predominantly class I- and class II-restricted response, respectively. The mice which had been immunized with the secreted form of NP show increased helper T cell (i.e. predominantly class II-restricted) responses when compared to immunization with the gene encoding the non-secreted antigen. Thus, addition of secretory signal sequence to the antigenic gene produces an increased helper T cell response (i.e. predominantly a class II-restricted response).

### Examples of delivery systems that allow presentation of antigen in association with Class I and Class II MHC (in vitro).

The following examples provide *in vitro* assays for differentiating MHC Class I and Class II immune responses, and for obtaining an increased MHC Class II immune response using constructs and methods according to the invention.

It has been shown that dendritic cells (DC) can be derived from Peripheral Blood Mononuclear Cells (PBMCs) *in vitro* by culturing with GM-CSF and IL-4 for one week (Romain *et al*. (1994) *J. Exp. Med.* 180:83), and that these cells can then be matured resulting in an increase in their ability to stimulate naive allogenic T cells and a decrease in their capacity to process antigen (Sallusto *et al*. (1994) *J. Exp. Med*. 179: 1109). This *in vitro* system has been utilised to assess the ability of the delivery vehicle to deliver antigens encoding genes and to assess their presentation by Class I or Class II MHC molecules.

### Transfection of Dendritic Cells

To demonstrate that a complex according to the invention is able to transfect the in vitro matured dendritic cells (DC) described above, DC were transfected with peptide complexes containing pEGFP-N1 (commercially available plasmid from Clonetech). The plasmid, pEGFP-N1 encodes green flourescent protein (GFP) which can be visualised by its natural flourescence when visiualised via a flourescence microscope. The peptide complexes were made with 2 ug NBC9 and 0.6 ug LIP 9 per ug of pEGF-N1 (Lip 9 is a N-palmityl derivative of NBC9 as described in International Patent Application No. PCT/GB96/01396). The complexes were made up by adding the following constituents in the following order mixing the sample well after each addition:

| | |
|---|---|
| NBC 9 (10 mg/ml in water) | 43.75 ml |
| Sterile milli-Q filtered water | 507.5 ml |
| 0.5 M phosphate buffer, pH 7.4 | 43.75 ml |
| 5M NaCl | 105 ml |
| pEGFP-N1 (0.5 mg/ml) | 175 ml |
| Lip 9 (1 mg/ml) | 52.9 ml |

The mixture was incubated at room temperature for 1 hour and overnight at 4°C. 850 ml was then diluted into 3400 ml of PEG dilution buffer.

| PEG dilution buffer | |
|---|---|
| | 10% PEG 8000 |
| | 25 mM phosphate pH 7.4 |
| | 37.5 mM NaCl |

DC were generated as described by Romani et al (J. Exp. Med. 1994 180:83- 93). Briefly, PBMC were prepared from Buffy coats by standard protocols known in the art. The PBMC were resuspended in RPMI-1640, 10% FCS and were allowed to adhere to plastic dishes. After 2 hours at 37°C, 5% CO₂ the nonadherent cells were removed, the adherent cells were gently washed and subsequently cultured with GM-CSF (800 U/ml) and IL-4 (500 U/ml). The cultures were fed with cytokines every second day of culture.

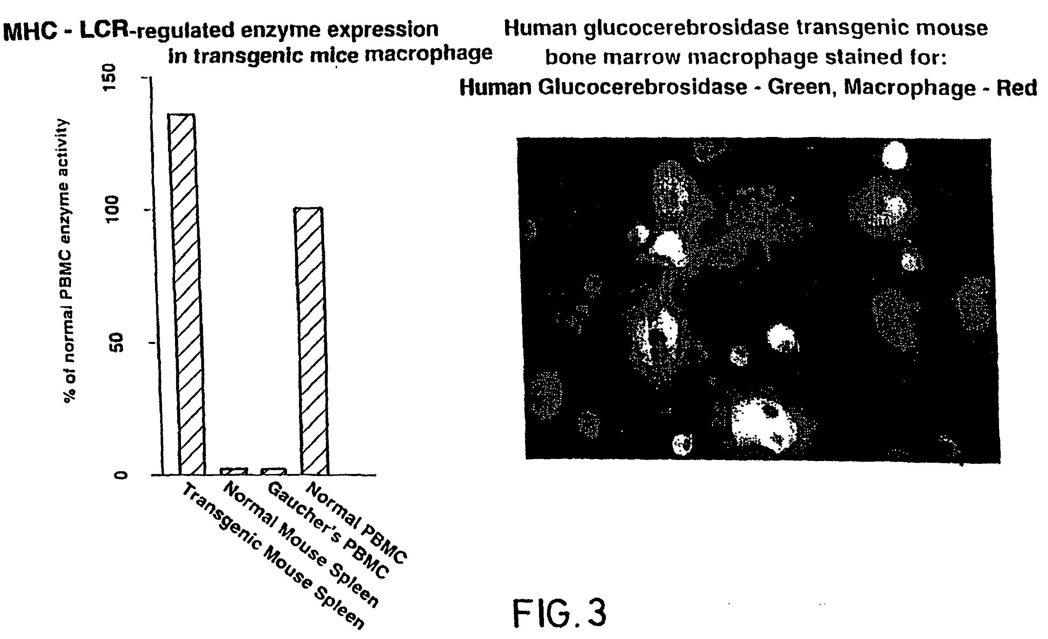

On day 7 of the culture the cells were harvested and resuspended to 1.26 x 106 cells/ml in RAT medium (RPMI 1640, human serum albumin 1 mg/ml; human transferrin (partly saturated) 50 ug/ml). Using 6 well plates 12 ul of 10 mM chloroquine and 863 ul of DC were added to a well. 125 ul of complex was then added per well and the cells were incubated at 37°C, 5% CO2 for 4 hours. The cells were removed from the wells harvested and resuspended in 1 ml complete medium (RPMI-1640, 10% FCS) per well and return to the plate for a 24 hour incubation at 37°C, 5% CO2. Each well was then harvested, cytospun onto a slide and tranfected cells were then visualised using a LEICA flourescence microscope (positive cells appear green). Figure 3 shows a typical field of view.

### To demonstrate that cells can be transfected with complexes that contain antigen and thus antigen and DNA can be delivered to the same cell.

COS cells have been transfected with complexes consisting of K6ClII peptide and pCMVb (Clonetech) whereas COS cells incubated with an equivalent amount of naked DNA show no transfection.

K6ClII is a peptide that contains an epitope of influenza A nucleoprotein (A/NT/60/68), and a stretch of lysine residues for ionic interaction with the phosphate backbone of the DNA. Thus this peptide is an antigen-DNA binding fusion peptide. The sequence of the peptide is:

NH2-KKKKKKGGFLGFWRGENGRKTRSAYERMCNILKGK-COOH

The DNA and complex solutions were made as follows: DNA solution: 100 ug/ml pCMVb, 25 mM phosphate pH 7.4, 0.6 M NaCl. Complex solution: 100 ug/ml pCMVb, 25 mM phosphate pH 7.4, 0.6 M NaCl, 200 ug/ml K6ClII. These solutions were incubated at room temperature for 1 hour and then 4°C overnight. COS cells were seeded at 1x10⁵ cells per well in 6 well plates (in DMEM, 10% Fetal calf serum (FCS)) 24 hours before incubation with complex or DNA.

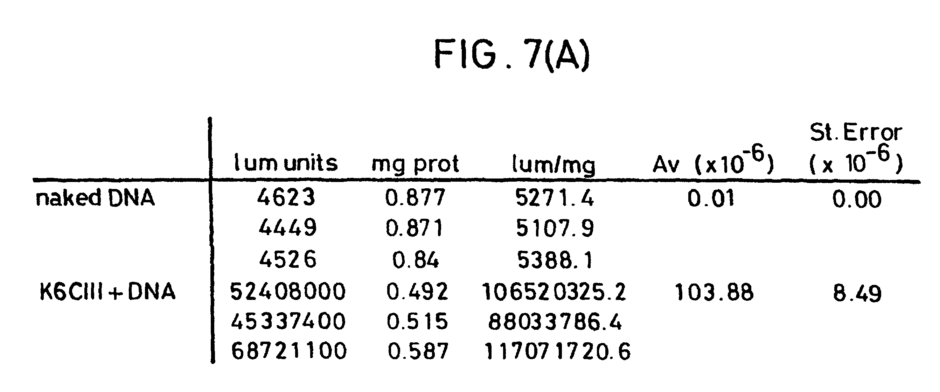

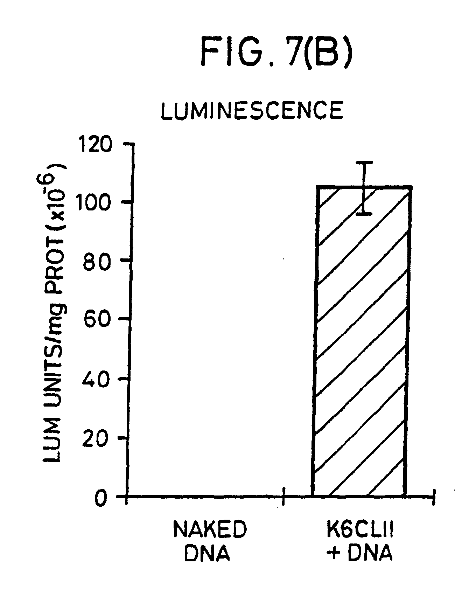

The DNA and complex solutions were diluted into PEG diluent (10% PEG 8000, 25 mM phosphate pH 7.4, 37.5 mM NaCl) to a final DNA concentration of 20 ug/ml. Just prior to transfection the COS cells were washed with PBS and 875 ul RATQ medium (RAT medium with 137.14 uM chloroquine) was added per well. 125 ul of diluted complex or DNA solution was then added per well and the cells were incubated at 37°C for 5 hours. The cells were then assayed for b-galactosidase activity using the Tropix Galacto-Light™ kit (Tropix, catalogue no. BL300G) according to the manufacturers instructions. Briefly, the wells were washed with DMEM, 10% FCS, and then 250 ul of lysis buffer (provided by the kit, with dithiothreitol added to 1 mM) was added per well. The cells were then scraped off and were pipetted up and down to aid cell lysis. The lysed cells were transfered to an eppendorf tube and centrifuged at 13K for 2 mins. The supernantant was transferred to a clean eppendorf tube. 2-10 ul of the supernantant was added to a luminometer assay tube and was made up to 10 ul with lysis buffer. 100ml of Reaction buffer is added to each tube, the samples are incubated at room temperature for 60 mins and then analysed using Light Emission Accelerator and a Barthoid lumat LB 9501 luminometer. The protein concentration of the cleared cell lysate was measured using the Bio-Rad DC protein assay kit (Bio-Rad). The results are then expressed as relative light units per mg of protein (Figure 7). Each transfection was carried out in triplicate.

Clearly, there is no transfection of the COS cells with naked DNA, but there is very efficient transfection with K6ClII/DNA complex. Since it is known in the art that cationic peptide/DNA complexes are taken up into cells through the endocytic pathway (Wagner, E., Curiel, D. and Cotten, Matt (1994) Advanced Drug Delivery Reviews 14: 113-135) the epitope containing K6ClII peptide must be delivered to the endosomes of the transfected cells. The endosome is the compartment of the APC delivery to which results in Class II antigen presentation.

### To demonstrate that DC can be transfected with complexes that contain antigen.

Complexes are made which are similar to those described in the previous example. These complexes may contain DNA encoding a reporter gene (e.g. luciferase) which is condensed by a peptide/protein which contains an antigenic sequence and a DNA-binding sequence, such as K6ClII. The complexes may also include other DNA condensing peptides or other lipidated peptides as described in International Patent Application No.PCT/GB96/01396. Such complexes have been shown to transfect COS cells (see above) and experimental optimisation of the complexes will result in transfection of DC. Optimisation can be done by empirically testing slightly different complexes for their ability to transfect DC. The transfection efficiency can be assessed by methods such as visualising green flourescent protein (GFP) expressing cells using a flourescence microscope or measuring luciferase or b-galactosidase activity. Methodologies for measuring these reporter proteins are described herein.

### To demonstrate that antigen which is incubated with cells when complexed into a delivery vehicle is more readily cell associated than free antigen.

The antigen used in these experiments is a peptide that contains an epitope sequence from influenza A nucleoprotein (K6CLII), as above. The peptide was flourescently labelled through the cysteine using Fluroscein-maleimide. The labelling was approximately 7% efficient. The protocol for the labelling was as follows: 4.2 mg K6CLII was dissolved in 0.5 ml 20 mM MES, pH 6.5. Next, 2.1 mg fluorescein-5-maleimide (Molecular Probes, Europe) was dissolved in 250 ul ethanol and added to the peptide solution. Ethanol was added dropwise until the agitated solution cleared. After 1 h at 24°C the sample was desalted on a PD-10 column (Pierce & Warriner, UK) using 50% aqueous acetonitrile. The fractions containing labelled peptide were lyophilised and stored at -20°C. This peptide will be termed K6ClII-F1.

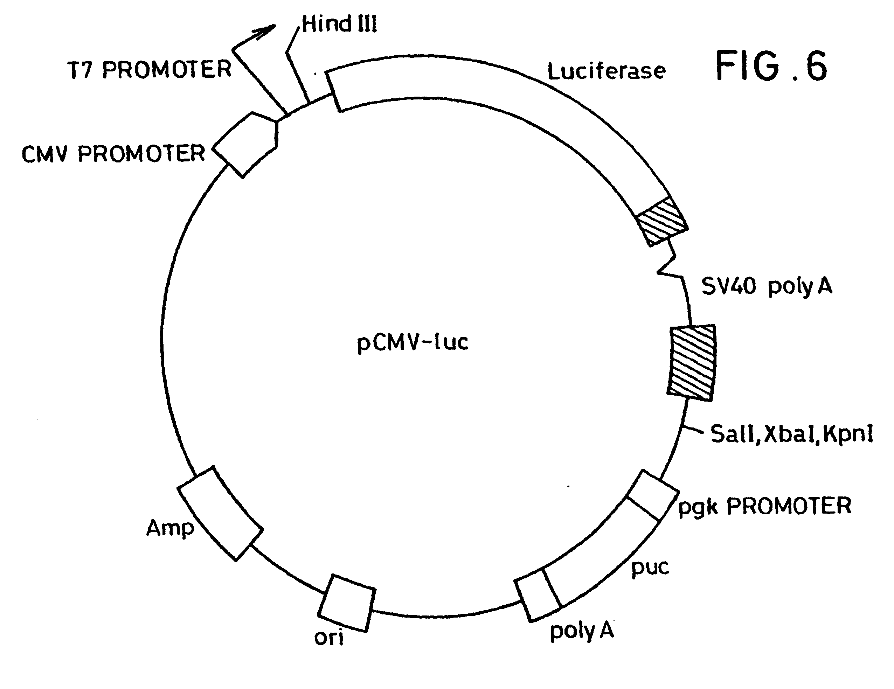

A non-viral delivery system was made using K6ClII-fℓ and pCMVluc (Figure 6). The delivery vehicle was made in the following way. Two separate solutions were made up peptide and DNA Peptide solution: 19.2 ml of K6ClII-F (@ 5 mg/ml in sterile water) was added to 1180.8 ml of 10 mM HEPES, 150 mM NaCl (HBS). DNA solution: 56.5 ml of pCMVluc (@ 0.5 mg/ml) was added to 1143.5 ml of HBS.

The peptide solution was then flash mixed with the DNA solution by adding quickly by pipette followed by repeated pipetting. The complex solution was left at room temperature for 1-2 hours before adding to the cells.

A control peptide solution contained 19.2 ml of K6ClII-F1 (@ 5 mg/ml) was added to 2380.8 ml of 10 mM HEPES, 150 mM NaCl (HBS).

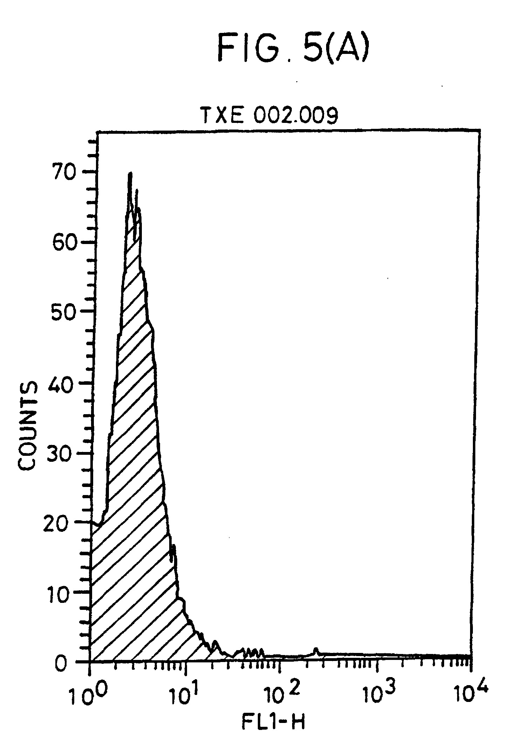

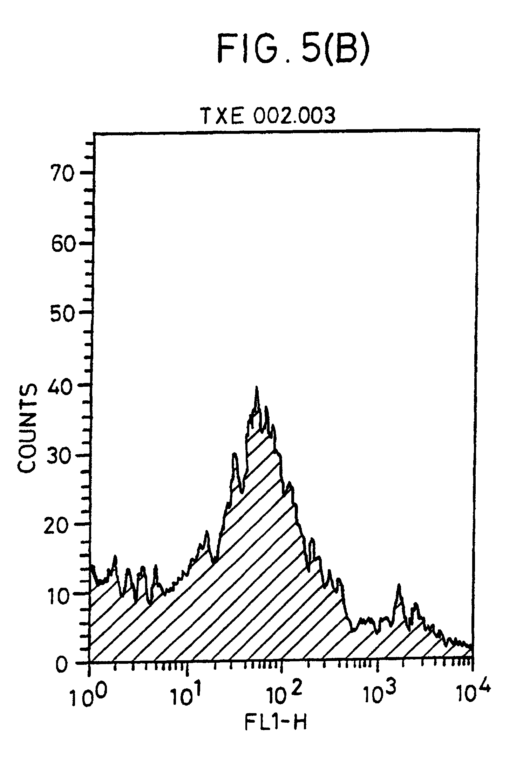

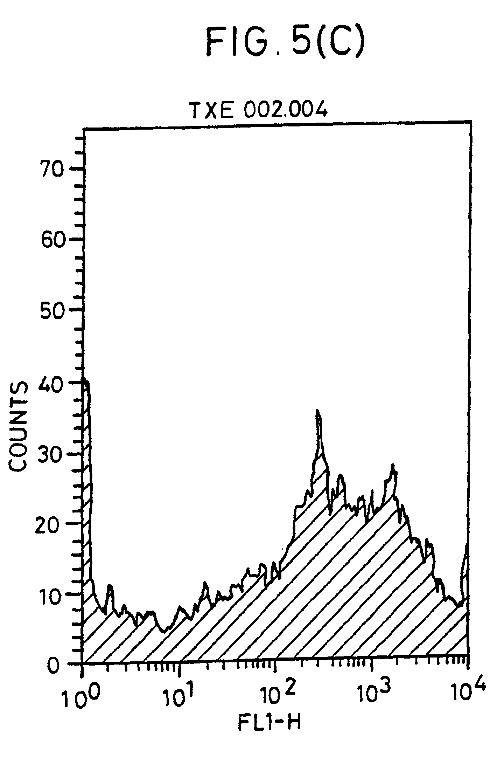

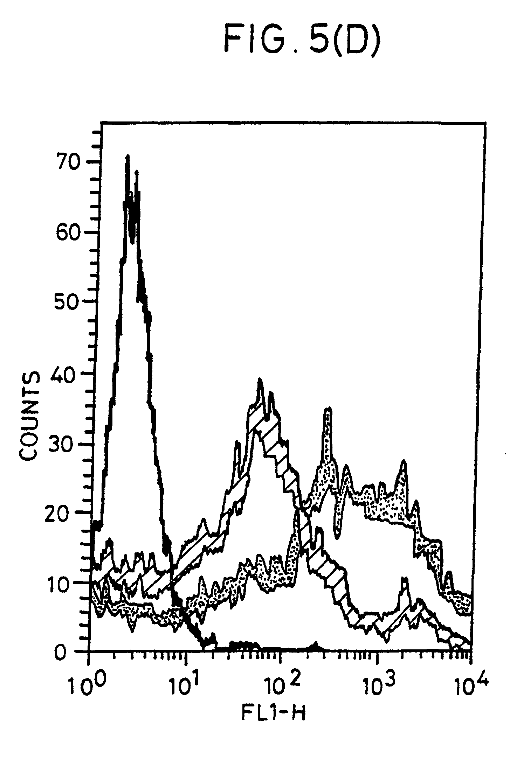

COS cells were seeded at 1x10⁵ cells per well in 6 well plates 24 hours before incubation with complex, peptide or buffer. The COS cells were washed once with PBS and 800 ml RAT medium was added. 200 ml of complex, free peptide or HBS were then added to the cells. After an incubation of 30 mins the cells were washed twice with PBS and then incubated with 2 ml PBS, 1 mM EDTA to release the adhered cells. The cells were harvested, resuspended in 500 ml PBS and analysed by FACS analysis (Beckton Dickinson FACScan). The FACS data clearly show that the cells to which complex has been delivered are associated with more fluorescence and therefore more peptide (i.e. antigen) than those incubated with peptide alone (Figure 5).

### To demonstrate that DC transfected with antigen expressing constructs can stimulate autologous T cells.

It has previously been shown that transfected DC are capable of stimulating the relevant T cell clones (Alijagic et al. Eur. J. Immunol. 1995 25:3100-3107).

Human PBMC were prepared from Buffy coats by methods known in the art (Current Protocols in Immunology, ed. J.E. Coligan et al). The PBMCs are split into two portions, from one T cells were purified by standard non- T cell depletion (magnetic beads) technologies known in the art (Current Protocols in Immunology, ed. J.E. Coligan et al) and from the other portion DC were derived as described above.

The DC were then transfected with plasmid encoding influenza A nucleoprotein protein by similar complexes to those described above. The complexes require optimisation to increase transfection efficiency; as described in PCT/GB96/01396. The transfected DC were then incubated with the autologous T cells at various ratios and the proliferative response of the T cells was measured by 3H-thymidine incorporation by methodolgy known to those familiar with the art (Current Protocols in Immunology, ed. J.E. Coligan et al). In the presence of transfected DC the T cell proliferation is expected to be substantially greater than when incubated with untransfected DC.

There are a number of methods by which presentation of influenza nucleoprotein (NP) can be analysed to assess whether it is Class I or II restricted. The ability of the DC to stimulate CD4+ or CD8+ subsets of the T cell population can be measured. Transfected DC are incubated at different DC:T cell ratios with either autologous CD4+ or CD8+ T cells. The relative proliferation of the T cells then allows assessment of whether the antigen (NP) is being presented in a Class I or Class II restricted manner. Stimulation of CD4+ cells signifies Class II presentation while stimulation of CD8+ cells signifies Class I presentation.

After transfection of the DC with an NP expressing plasmid the DC will be capable of stimulating CD8+ cells to proliferate to a greater extent than CD4+ cells and thus antigen presentation was mainly MHC Class I restricted. Class I or II restriction of antigen presentation can also be measured by analysis of the cytokines produced when transfected DC are incubated with autologous T cells. Transfected DC and autologous T cells are incubated together at different ratios (usually in the range of 1:1 to 1:100 of DC:T cell).

### To demonstrate that antigen delivered to DC is presented to autologous T cells and that presentation is principally via MHC Class II presentation.

Antigen can be delivered to DC by inclusion of the antigen in the delivery vehicle. There are a number of methods by which antigen may be included in the delivery system. The antigen may be conjugated, by conjugation chemistry, to DNA-binding peptides. The conjugation chemistries may include, but are not restricted to, those described in International Patent Application No. PCT/GB96/01396. The antigen may be an epitope which, together with a DNA-binding sequence, can be synthesised by standard peptide chemistry techniques. The antigen may also be a recombinant molecule which is designed to contain the antigen and a single, or multiple, DNA-binding sequences. In the above cases the antigen is bound to the complex by ionic interaction between the DNA-binding function and the phosphate backbone of the DNA. The antigen-DNA-binding sequence conjugate may or may not be the only DNA condensing agent in the delivery vehicle. The antigen may also be bound to the delivery vehicle by hydrophobic, electrostatic, covalent, or other interactions. The antigen may be any protein, or part thereof, to which an immune response can be generated.

Delivery vehicles that contain antigen may be tested for their efficiency of delivery of antigen to DC by testing, as follows. Test complexes are made and tested for their ability to deliver antigen to DC by measuring presentation of the antigen by the DC to which antigen has been delivered. Methods to measure the presentation of the antigen are described below. Methods by which the delivery may be optimized for better nucleic acid/antigen delivery are described in International Patent Application No. PCT/GB96/01396.

Using the complex it can then be shown that DC that have been incubated with antigen containing delivery vehicle are able to present the antigen to T cells. The antigen used in these experiments must be a 'Recall antigen'. A Recall antigen is an antigen to which the donor from whom the DC and T cells were purified has already generated an immune response. Examples of recall antigens are tetanus toxoid and influenza nucleoprotein (in donors who have previously been infected with influenza). Presentation of the recall antigen can be assessed by incubation of DC with autologous T cells and measurement of the proliferation of the T cells by measuring 3H-thymidine incorporation. The DC and T cells are incubated at various ratios (usually 1:1 to 1:100 DC:T cells). To test whether the antigen is presented by Class I or Class II MHC molecules one of a number of assays can be carried out. These include, but are not restricted to, measurement of the ability of the DC to stimulate CD4+ or CD8+ subsets of the T cell population or measurement of cytokine production when DC are incubated with autologous T cells. To measure of the ability of the DC to stimulate CD4+ or CD8+ subsets of the T cell population transfected DC are incubated at different DC:T cell ratios with either autologous CD4+ or CD8+ T cells. The relative proliferation of the Tcells then allows assessment of whether the antigen is being presented in a Class I or Class II restricted manner. Stimulation of CD4+ cells signifies Class II presentation while stimulation of CD8+ cells signifies Class I
presentation.

Similar assays are set up to measure cytokine production. DC are incubated with autologous T cells and after a given time point, usually but not always 24 hrs samples of the medium are taken and assayed for cytokines. Examples of cyokines which are commonly measured are IFN-gamma (signfies a Th1 response) or IL-4 (signifies Th2 response). Commercial kits are available to measure the levels of these cytokines (R&D Systems). Cytokine production can also be measured by more sensitive assays based on the ELISPOT technique (Current Protocols in Immunology, ed. J.E. Coligan et al).

### To demonstrate that delivery of antigen and DNA to DC generates a stronger proliferative response from autologous T cells than delivery of antigen or DNA alone

Delivery of antigen alone or DNA alone to DC generally leads to presentation of epitopes of the antigen, in association with MHC molecules, on the surface of the DC. Delivery of antigen tends to lead to presentation in association with MHC Class II molecules, whereas delivery of DNA (i.e. transfection of the DC) generally leads to presentation in association with Class I molecules. Delivery of both DNA and antigen leads to increased presentation by the DC compared to delivering either DNA or antigen alone.

Delivery vehicles that contain antigen and DNA may be tested for optimal delivery to a target APC cell as follows. Test delivery vehicles are made and tested for their ability to deliver antigen and DNA to DC by measuring presentation of the antigen by the DC to which antigen has been delivered. Methods to measure the presentation of the antigen are described in the two previous examples. Methods by which the complex may be optimized for better delivery are described in International Patent Application No. PCT/GB96/01396.

DC to which both DNA and antigen are delivered are expected to show increased ability to stimulate proliferation of autologous T cells (measured by ³H-thymidine incorporation) compared to DC to which only DNA or protein/peptide antigen has been delivered. Thus, delivery of both DNA and peptide/protein antigen is expected to show significant advantage for the generation of an immune response compared with delivery of either separately.

The invention also encompasses the co-administration of two separate moieties, (i) nucleic acid and (ii)peptide antigen, as a mixture. It is believed that such co-administration will result in an increased ability of the APC to stimulate proliferation of autologous T cells compared to APC to which only DNA or protein/peptide antigen has been delivered.

### Addition of secretion signal to antigen gene

To demonstrate that transfection with a delivery vehicle containing DNA encoding an antigen gene with a secretion signal sequence that is functional in mammalian cells gives rise to a greater Class II response than transfection with a delivery vehicle in which an non-secreted version of the antigen is produced, *in vitro* derived dendritic cells (from donors previously infected with influenza A) are transfected with a delivery vehicle containing a DNA molecule encoding the influenza nucleoprotein gene with or without a secretory signal sequence. Syngeneic T cells are then assayed for class I-and class II-restricted responses when incubated with the transfected DCs (prepared from the same PBMC sample as the transfected DCs). Dendritic cells transfected with the secreted form of NP show increased Class II-restricted T cell activation ability when compared to DCs transfected with the gene encoding the non-secreted antigen. Thus, addition of secretory signal sequence to the antigenic gene produces an increased class II-restricted response.

### Comparative Demonstration that a Complex or Mixture According to the Invention, Containing an Epitope and a Nucleic Acid, Induces a Stronger Immune Response than a Free Epitope.

To demonstrate that delivery of an epitope or antigen as part of a delivery complex is more effective in inducing immunogenicity than delivery of 'free' antigen (that is, an epitope or antigen such as a peptide or polypeptide that is free of nucleic acid), mice are vaccinated with a mixture or complex according to the invention, i.e., nucleic acid and a peptide antigen, or with the same peptide antigen free of nucleic acid. The immune responses in the vaccinated mice are measured by standard immunological assays which are well known to those skilled in the art, such assays are Cr⁵¹ release assays, T cell proliferation assays and cytokine assays (Current Protocols in Immunology, ed. J.E. Coligan et al), as described herein.

For example, a comparative demonstration may be performed using an antigen which consists of an epitope of an antigenic protein known to be presented by the mouse strain being immunized, such as a peptide epitope of influenza nucleoprotein, or it can be a whole protein, such as the influenza nucleoprotein. The mice are vaccinated by subcutaneous, intravenous, intraperitoneal or other routes. The mice may be subject to boosting injections at periods of 1 to 4 weeks after the priming injections. The mice are sacrificed at least 3 weeks after the priming or boosting injection, their splenocytes are harvested and subject to standard immunological assays to determine the level of the immune response. A complex or mixture according to the invention is determined to elicit a more effective immune response where the proliferative response to the test antigen of splenocytes from mice immunized with a complex or mixture according to the invention is at least two-fold and preferably five- to ten-fold greater in magnitude than the proliferative response of splenocytes from animals immunized with antigen alone.

Once the magnitude of the immune response is determined to be at least two-fold greater in a comparative assay, the type of immune response generated is further defined using Cr⁵¹ release assays and cytokine assays. Complexes or mixtures according to the invention will preferably elicit both a cellular and a humoral immune response, at least one of which is two-fold or greater in magnitude than a control or prior art vaccine in a comparative assay. Alternatively, the inventive complexes or mixtures will elicit either a cellular or a humoral immune response, that response of which is at least two-fold and preferably five- to ten-fold greater in magnitude than a control vaccine in a comparative assay. A cellular immune response is indicated by a cytotoxic T cell response, as measured by Cr⁵¹ release assays, and is indicative of a class I restricted response. Measurement of this response in the presence of anti-murine CD4 versus anti-murine CD8 antibodies may provide a more accurate indication of the MHC restriction of the response, and whether the immune response also involves a humoral immune response. That is, a response in the presence of anti-CD8 antibodies is Class II restricted whereas a response in the presence of anti-CD4 antibodies is Class I restricted. Anti-CD4 or anti-CD8 antibodies also may be used in the proliferation assays to indicate whether the immune response is MHC class I or MHC class II restricted.

### OTHER EMBODIMENTS

Other embodiments are within the following claims.

## Claims

1. A vaccine composition comprising a mixture of a nucleic acid encoding a first epitope and a peptide comprising a second epitope wherein the mixture does not comprise a whole pathogen **characterised in that** the composition alters the immune response against a disease when administered to a mammal.

2. A vaccine composition of claim 1 wherein the composition comprises a complex of a nucleic acid encoding said first epitope and a peptide comprising said second epitope.

3. A vaccine composition according to claim 1 or claim 2 comprising a recombinant nucleic acid encoding a first epitope and a peptide comprising a second epitope that is not normally present in antigen presenting cells.

4. A vaccine composition of any of claims 1 to 3 wherein said first and said second epitopes are the same epitope.

5. A vaccine composition of any of claims 1 to 3 wherein said first and said second epitopes are different epitopes.

6. A vaccine composition of claim 5 wherein said first and said second epitopes are epitopes of a single polypeptide.

7. A vaccine composition of claim 5 wherein said first epitope is an epitope of a first polypeptide and said second epitope is an epitope of a second polypeptide.

8. A vaccine composition of any of claims 5 to 7 wherein said first and said second epitopes are epitopes of a single organism.

9. A vaccine composition of any of the preceding claims wherein the nucleic acid encodes more than one epitope.

10. A vaccine composition of any of the preceding claims wherein the peptide comprises more than one epitope.

11. A vaccine composition of any of the preceding claims wherein said first and/or second epitope is from an infectious agent.

12. A vaccine composition of any of the preceding claims wherein said first and/or second epitope is present in the mammal during the course of a disease.

13. A vaccine composition of claim 12 wherein the disease is caused by an infectious pathogen and said nucleic acid comprises an open reading frame from the pathogen's genome.

14. A vaccine composition of claim 13 wherein said nucleic acid comprises multiple open reading frames from the pathogen's genome.

15. A vaccine composition of any of the preceding claims wherein the nucleic acid comprises a mufti-gene construct.

16. A vaccine composition of claim 15 wherein the genes of said multi-gene construct are co-ordinately expressed.

17. A vaccine composition according to any of the preceding claims wherein said nucleic acid encodes said first epitope and a second epitope, and said first epitope is present in a mammal during the course of a first disease and said second epitope is present in a mammal during the course of a second disease.

18. A vaccine composition of claim 17 wherein said first disease is caused by a first pathogen and said second disease is caused by a second pathogen.

19. A vaccine composition according to any of the preceding claims wherein said second epitope is present in a two-domain polypeptide comprising said second epitope fused to a nucleic acid-binding amino acid sequence.

20. A vaccine composition of claim 19 wherein said second epitope is from the same organism as the nucleic acid-encoded epitope.

21. A vaccine composition of any of claims 19 to 20 wherein said second epitope is within a polypeptide sequence containing multiple epitopes.

22. A vaccine composition of any of the preceding claims wherein said first and/or said second epitope comprises an immunodominant epitope of influenza NP.

23. A vaccine composition of any of the preceding claims, wherein said complex is adapted for selective expression in antigen presenting cells.

24. A vaccine composition of any of the preceding claims, said complex further comprising a cell-targeting ligand for targeting professional antigen presenting cells.

25. A vaccine composition of any of claims 1 to 23, said complex further comprising a gene control element for obtaining selective expression in antigen presenting cells.

26. A vaccine composition of claim 25 wherein said nucleic acid further comprises a locus control region which restricts expression of a coding sequence that is operatively linked thereto to professional antigen presenting cells.

27. A vaccine composition of claim 26, wherein the locus control region is the MHC class II LCR or the Ig LCR.

28. A vaccine composition according to any of claims 1 to 27 comprising a complex of a nucleic acid and a peptide comprising at least one epitope to which an immune response is elicited.

29. A vaccine composition of claim 28 wherein at least one epitope is from an infectious agent or an organism.

30. A vaccine composition of any of claims 28 to claim 29 wherein at least one epitope is present in the mammal during the course of a disease.

31. A vaccine composition of claim any of claims 28 to 30 wherein the peptide comprises more than one epitope and the epitopes are epitopes of the same antigen.

32. A vaccine composition of claim 31 wherein said epitopes are epitopes of the same infectious agent or organism.

33. A vaccine composition of any of claims 31 to 32 wherein said epitopes comprise an immunodominant epitope of influenza NP.

34. A vaccine composition of any of claims 28 to 33 wherein at least one epitope is present in a two-domain polypeptide comprising said epitope fused to a nucleic acid-binding amino acid sequence.

35. A vaccine composition of any of claims 28 to 34 said complex further comprising a cell-targeting ligand for targeting professional antigen presenting cells.

36. A vaccine composition according to any of claims 1 to 27, further comprising a vector comprising a nucleic acid encoding at least one epitope and a sequence which permits maintenance of said vector in episomal form.

37. A vaccine composition of claim 36, wherein said sequence which permits maintenance of said vector in episomal form comprises a papilloma virus sequence.

38. A vaccine composition of claim 37 wherein said papilloma virus sequence comprises a minimal origin of replication and a minimal maintenance element.

39. A vaccine composition of claim 37 or claim 38 wherein said papilloma virus sequence further comprises the E1 and E2 genes.

40. A vaccine composition of any of claims 37 to 39 wherein said papilloma virus sequence is from bovine papilloma virus.

41. A composition according to any of claims 1 to 40 for use as a medicament.

## Patentansprüche

1. Eine Impfstoffzusammensetzung, bestehend aus einer Mischung einer ein erstes Epitop kodierenden Nukleinsäure und eines Peptids, das aus einem zweiten Epitop besteht, wobei die Mischung nicht aus einem ganzen Krankheitserreger besteht, **dadurch gekennzeichnet, dass** die Zusammensetzung bei der Abgabe an einen Säuger die Immunantwort auf eine Krankheit verändert.

2. Impfstoffzusammensetzung gemäß Anspruch 1, wobei die Zusammensetzung aus einem Komplex aus einer Nukleinsäure, die dieses erste Epitop kodiert und einem Peptid, das aus dem zweiten Epitop besteht, besteht.

3. Impfstoffzusammensetzung gemäß Anspruch 1 oder Anspruch 2, bestehend aus einer rekombinanten Nukleinsäure, die ein erstes Epitop kodiert, und einem Peptid, das aus einem zweiten Epitop besteht, das normalerweise nicht in antigenpräsentierenden Zellen vorhanden ist.

4. Impfstoffzusammensetzung gemäß einem der Ansprüche 1 bis 3, wobei das erste und das zweite Epitop dasselbe Epitop sind.

5. Impfstoffzusammensetzung gemäß einem der Ansprüche 1 bis 3, wobei das erste und das zweite Epitop verschiedene Epitope sind.

6. Impfstoffzusammensetzung gemäß Anspruch 5, wobei das erste und das zweite Epitop Epitope eines einzelnen Polypeptids sind.

7. Impfstoffzusammensetzung gemäß Anspruch 5, wobei das erste Epitop ein Epitop eines ersten Polypeptids ist und das zweite Epitop ein Epitop eines zweiten Polypeptids ist.

8. Impfstoffzusammensetzung gemäß einem der Ansprüche 5 bis 7, wobei das erste und das zweite Epitop Epitope eines einzelnen Organismus sind.

9. Impfstoffzusammensetzung gemäß einem der vorhergehenden Ansprüche, wobei die Nukleinsäure mehr als ein Epitop kodiert.

10. Impfstoffzusammensetzung gemäß einem der vorhergehenden Ansprüche, wobei das Peptid aus mehr als einem Epitop besteht.

11. Impfstoffzusammensetzung gemäß einem der vorhergehenden Ansprüche, wobei das erste und/oder das zweite Epitop von einer infektiösen Einheit stammt.

12. Impfstoffzusammensetzung gemäß einem der vorhergehenden Ansprüche, wobei das erste und/oder das zweite Epitop während des Verlaufs der Krankheit in dem Säuger vorhanden sind.

13. Impfstoffzusammensetzung gemäß Anspruch 12, wobei die Krankheit durch einen infektiösen Krankheitserreger verursacht wird und wobei die Nukleinsäure aus einem offenen Leserahmen von dem Genom des Krankheitserregers besteht.

14. Impfstoffzusammensetzung gemäß Anspruch 13, wobei die Nukleinsäure aus mehreren offenen Leserahmen von dem Genom des Krankheitserregers besteht.

15. Impfstoffzusammensetzung gemäß einem der vorhergehenden Ansprüche, wobei die Nukleinsäure aus einem Multigen-Rekombinationsprodukt besteht.

16. Impfstoffzusammensetzung gemäß Anspruch 15, wobei die Gene dieses Multigen-Rekombinationsproduktes koordiniert exprimiert werden.

17. Impfstoffzusammensetzung gemäß einem der vorhergehenden Ansprüche, wobei die Nukleinsäure das erste Epitop und ein zweites Epitop kodiert, und wobei das erste Epitop während des Verlaufs einer ersten Krankheit in dem Säuger vorhanden ist und wobei das zweite Epitop während des Verlaufs einer zweiten Krankheit in einem Säuger vorhanden ist.

18. Impfstoffzusammensetzung gemäß Anspruch 17, wobei die erste Krankheit durch einen ersten Krankheitserreger verursacht wird und die zweite Krankheit durch einen zweiten Krankheitserreger verursacht wird.

19. Impfstoffzusammensetzung gemäß einem der vorhergehenden Ansprüche, wobei das zweite Epitop in einem Zwei-Domänen-Polypeptid vorhanden ist, wobei das zweite Epitop mit einer nukleinsäurebindenden Aminosäuresequenz verschmolzen ist.

20. Impfstoffzusammensetzung gemäß Anspruch 19, wobei das zweite Epitop von demselben Organismus wie das nukleinsäurekodierte Epitop stammt.

21. Impfstoffzusammensetzung gemäß einem der Ansprüche 19 bis 20, wobei das zweite Epitop innerhalb einer mehrere Epitope enthaltenden Polypeptidsequenz liegt.

22. Impfstoffzusammensetzung gemäß einem der vorhergehenden Ansprüche, wobei das erste und/oder das zweite Epitop aus einem immundominanten Epitop von Influenza NP besteht.

23. Impfstoffzusammensetzung gemäß einem der vorhergehenden Ansprüche, wobei der Komplex für die selektive Expression in antigenpräsentierenden Zellen angepasst ist.

24. Impfstoffzusammensetzung gemäß einem der vorhergehenden Ansprüche, wobei der Komplex ferner aus einem auf Zellen zielenden Ligand zum Zielen auf professionelle antigenpräsentierende Zellen besteht.

25. Impfstoffzusammensetzung gemäß einem der Ansprüche 1 bis 23, wobei der Komplex ferner aus einem Genkontrollelement zur selektiven Expression in antigenpräsentierenden Zellen besteht.

26. Impfstoffzusammensetzung gemäß Anspruch 25, wobei die Nukleinsäure ferner aus einer Locus-Kontroll-Region, die die Expression einer kodierenden Sequenz, die operativ mit dieser verbunden ist, auf professionelle antigenpräsentierende Zellen einschränkt.

27. Impfstoffzusammensetzung gemäß Anspruch 26, wobei die Locus-Kontroll-Region die LCR der MHC Klasse II oder die LCR von Ig ist.

28. Impfstoffzusammensetzung gemäß einem der Ansprüche 1 bis 27, bestehend aus einem Komplex einer Nukleinsäure und eines Peptids, das aus mindestens einem eine Immunantwort auslösenden Epitop besteht.

29. Impfstoffzusammensetzung gemäß Anspruch 28, wobei mindestens ein Epitop aus einer infektiösen Einheit oder einem Organismus stammt.

30. Impfstoffzusammensetzung gemäß Anspruch 28 oder Anspruch 29, wobei mindestens ein Epitop während des Verlaufs einer Krankheit in dem Säuger vorhanden ist.

31. Impfstoffzusammensetzung gemäß einem der Ansprüche 28 bis 30, wobei das Peptid aus mehr als einem Epitop besteht und wobei die Epitope Epitope desselben Antigens sind.

32. Impfstoffzusammensetzung gemäß Anspruch 31, wobei diese Epitope Epitope derselben infektiösen Einheit oder desselben infektiösen Organismus sind.

33. Impfstoffzusammensetzung gemäß einem der Ansprüche 31 bis 32, wobei diese Epitope aus einem immundominanten Epitop von Influenza NP bestehen.

34. Impfstoffzusammensetzung gemäß einem der Ansprüche 28 bis 33, wobei mindestens ein Epitop in einem Zwei-Domänen-Polypeptid vorhanden ist, das aus einem mit einer nukleinsäurebindenden Aminosäuresequenz verschmolzenen Epitop besteht.

35. Impfstoffzusammensetzung gemäß einem der Ansprüche 28 bis 34, wobei der Komplex ferner aus einem auf Zellen zielenden Ligand zum Zielen auf professionelle antigenpräsentierende Zellen besteht.

36. Impfstoffzusammensetzung gemäß einem der Ansprüche 1 bis 27, ferner bestehend aus einem Vektor, der aus einer Nukleinsäure besteht, die mindestens ein Epitop und eine Sequenz kodiert, was die Erhaltung dieses Vektors in episomaler Form ermöglicht.

37. Impfstoffzusammensetzung gemäß Anspruch 36, wobei die Sequenz, die die Erhaltung des Vektors in episomaler Form ermöglicht, aus einer Papillomvirussequenz besteht.

38. Impfstoffzusammensetzung gemäß Anspruch 37, wobei die Papillomvirussequenz aus einem minimalen Replikationsursprung und einem minimalen Erhaltungselement besteht.

39. Impfstoffzusammensetzung gemäß Anspruch 37 oder Anspruch 38, wobei die Papillomvirussequenz ferner aus E1 und E2 Genen besteht.

40. Impfstoffzusammensetzung gemäß einem der Ansprüche 37 bis 39, wobei die Papillomvirussequenz von einem Rinderpapillomvirus stammt.

41. Zusammensetzung gemäß einem der Ansprüche 1 bis 40 zur Verwendung als Medikament.

## Revendications

1. Une composition de vaccin comprenant un mélange d'un acide nucléique codant pour un premier épitope et d'un peptide comprenant un deuxième épitope dans laquelle le mélange ne comprend pas d'agent pathogène entier **caractérisée en ce que** la composition modifie la réponse immunitaire à une maladie lorsqu'elle est administrée à un mammifère.

2. Une composition de vaccin de la revendication 1 dans laquelle la composition comprend un complexe d'un acide nucléique codant pour ledit premier épitope et un peptide comprenant ledit deuxième épitope.

3. Une composition de vaccin selon la revendication 1 ou la revendication 2 comprenant un acide nucléique recombiné codant pour un premier épitope et un peptide comprenant un deuxième épitope qui n'est normalement pas présent dans des cellules présentatrices d'antigènes.

4. Une composition de vaccin de n'importe lesquelles des revendications 1 à 3 dans laquelle ledit premier et ledit deuxième épitope sont le même épitope.

5. Une composition de vaccin de n'importe lesquelles des revendications 1 à 3 dans laquelle ledit premier et ledit deuxième épitope sont des épitopes différents.

6. Une composition de vaccin de la revendications 5 dans laquelle ledit premier et ledit deuxième épitope sont des épitopes d'un seul polypeptide.

7. Une composition de vaccin de la revendications 5 dans laquelle ledit premier épitope est un épitope d'un premier polypeptide et ledit deuxième épitope est un épitope d'un deuxième polypeptide.

8. Une composition de vaccin de n'importe lesquelles des revendications 5 à 7 dans laquelle ledit premier et ledit deuxième épitope sont des épitopes d'un seul organisme.

9. Une composition de vaccin de n'importe lesquelles des revendications précédentes dans laquelle l'acide nucléique code pour plus d'un épitope.

10. Une composition de vaccin de n'importe lesquelles des revendications précédentes dans laquelle le peptide comprend plus d'un épitope.

11. Une composition de vaccin de n'importe lesquelles des revendications précédentes dans laquelle ledit premier et/ou ledit deuxième épitope proviennent d'un agent infectieux.

12. Une composition de vaccin de n'importe lesquelles des revendications précédentes dans laquelle ledit premier et/ou ledit deuxième épitope sont présents dans le mammifère au cours de l'évolution d'une maladie.

13. Une composition de vaccin de la revendication 12 dans laquelle la maladie est causée par un agent pathogène infectieux et ledit acide nucléique comprend un cadre de lecture ouvert provenant du génome de l'agent pathogène.

14. Une composition de vaccin de la revendication 13 dans laquelle ledit acide nucléique comprend des cadres de lecture ouverts multiples provenant du génome de l'agent pathogène.

15. Une composition de vaccin de n'importe lesquelles des revendications précédentes dans laquelle l'acide nucléique comprend un produit de recombinaison multigénique.

16. Une composition de vaccin de la revendication 15 dans laquelle les gènes dudit produit de recombinaison multigénique sont exprimés de façon coordonnée.

17. Une composition de vaccin selon n'importe lesquelles des revendications précédentes dans laquelle ledit acide nucléique code pour ledit premier épitope et un deuxième épitope, et ledit premier épitope est présent dans un mammifère au cours de l'évolution d'une première maladie et ledit deuxième épitope est présent dans un mammifère au cours de l'évolution d'une deuxième maladie.

18. Une composition de vaccin de la revendication 17 dans laquelle ladite première maladie est causée par un premier agent pathogène et ladite deuxième maladie est causée par un deuxième agent pathogène.

19. Une composition de vaccin selon n'importe lesquelles des revendications précédentes dans laquelle ledit deuxième épitope est présent dans un polypeptide à deux domaines comprenant ledit deuxième épitope uni par fusion à une séquence aminoacide de liaison d'acide nucléique.

20. Une composition de vaccin de la revendication 19 dans laquelle ledit deuxième épitope provient du même organisme que l'épitope codé par l'acide nucléique.

21. Une composition de vaccin de n'importe lesquelles des revendications 19 à 20 dans laquelle ledit deuxième épitope se trouve à l'intérieur d'une séquence polypeptidique contenant des épitopes multiples.

22. Une composition de vaccin de n'importe lesquelles des revendications précédentes dans laquelle ledit premier et/ou ledit deuxième épitope comprend un épitope immunodominant d'influenza NP.

23. Une composition de vaccin de n'importe lesquelles des revendications précédentes, dans laquelle ledit complexe est adapté pour être exprimé de façon sélective dans des cellules présentatrices d'antigènes.

24. Une composition de vaccin de n'importe lesquelles des revendications précédentes, ledit complexe comprenant de plus un ligand de ciblage de cellules pour cibler des cellules présentatrices d'antigènes professionnelles.

25. Une composition de vaccin de n'importe lesquelles des revendications 1 à 23, ledit complexe comprenant de plus un élément de contrôle de gène pour obtenir une expression sélective dans des cellules présentatrices d'antigènes.

26. Une composition de vaccin de la revendication 25 dans laquelle ledit acide nucléique comprend de plus une région de contrôle de locus, laquelle restreint l'expression d'une séquence codante qui lui est fonctionnellement liée à des cellules présentatrices d'antigènes professionnelles.

27. Une composition de vaccin de la revendication 26, dans laquelle la région de contrôle de locus est le LCR du CMH de classe II ou le LCR Ig.

28. Une composition de vaccin selon n'importe lesquelles des revendications 1 à 27 comprenant un complexe d'un acide nucléique et d'un peptide comprenant au moins un épitope par rapport auquel une réponse immunitaire est déclenchée.

29. Une composition de vaccin de la revendication 28 dans laquelle au moins un épitope provient d'un agent infectieux ou d'un organisme.

30. Une composition de vaccin de n'importe lesquelles des revendications 28 à 29 dans laquelle au moins un épitope est présent dans le mammifère au cours de l'évolution d'une maladie.

31. Une composition de vaccin de n'importe lesquelles des revendications 28 à 30 dans laquelle le peptide comprend plus d'un épitope et les épitopes sont des épitopes du même antigène.

32. Une composition de vaccin de la revendication 31 dans laquelle lesdits épitopes sont des épitopes du même agent infectieux ou organisme.

33. Une composition de vaccin de n'importe lesquelles des revendications 31 à 32 dans laquelle lesdits épitopes consistent en un épitope immunodominant d'influenza NP.

34. Une composition de vaccin de n'importe lesquelles des revendications 28 à 33 dans laquelle au moins un épitope est présent dans un polypeptide à deux domaines comprenant ledit épitope uni par fusion à une séquence aminoacide de liaison d'acide nucléique.

35. Une composition de vaccin de n'importe lesquelles des revendications 28 à 34, ledit complexe comprenant de plus un ligand de ciblage de cellules pour cibler des cellules présentatrices d'antigènes professionnelles.

36. Une composition de vaccin selon n'importe lesquelles des revendications 1 à 27, comprenant de plus un vecteur consistant en un acide nucléique codant pour au moins un épitope et une séquence qui permet de maintenir ledit vecteur sous forme épisomale.

37. Une composition de vaccin de la revendication 36, dans laquelle ladite séquence qui permet de maintenir ledit vecteur sous forme épisomale consiste en une séquence de virus papilloma.

38. Une composition de vaccin de la revendication 37 dans laquelle ladite séquence de virus papilloma comprend une origine de réplication minimale et un élément de maintien minimal.

39. Une composition de vaccin de la revendication 37 ou de la revendication 38 dans laquelle ladite séquence de virus papilloma comprend de plus les gènes E1 et E2.

40. Une composition de vaccin de n'importe lesquelles des revendications 37 à 39 dans laquelle ladite séquence de virus papilloma provient du virus papilloma de bovin.

41. Une composition selon n'importe lesquelles des revendications 1 à 40 destinée à être utilisée comme médicament.
